# EUROPEAN PATENT APPLICATION

(11) **EP 4 541 784 A1**
(43) Date of publication of application: **23.04.2025**
(21) Application number: 23827210.8
(22) Date of filing: 20.06.2023
(51) Int. Cl.: C07C 211/63, B01J 29/70, B01J 35/10, C01B 39/48, C07B 61/00, C07C 1/24, C07C 11/04, C07C 11/06, C07C 11/08, C07C 209/60

(54) **QUATERNARY AMMONIUM SALT, ORGANIC STRUCTURE-DIRECTING AGENT, METHOD FOR PRODUCING QUATERNARY AMMONIUM SALT, CON-TYPE ZEOLITE, METHOD FOR PRODUCING CON-TYPE ZEOLITE, CATALYST, AND METHOD FOR PRODUCING LOWER OLEFIN**

(30) Priority: 20.06.2022 JP 2022098981; 20.06.2022 JP 2022098982; 20.06.2022 JP 2022098983
(71) Applicant: Mitsubishi Chemical Corporation, Tokyo 100-8251 (JP); Japan Technological Research Association Of Artificial Photosynthetic Chemical Process, Tokyo 113-8656 (JP)
(72) Inventor: SAITO, Yasuyo, Tokyo 100-8251 (JP); ONOZUKA, Hiroaki, Tokyo 100-8251 (JP); TSUTSUMINAI, Susumu, Tokyo 100-8251 (JP)
(74) Representative: Hoffmann Eitle
(86) International application number: PCT/JP2023/022837
(87) International publication number: WO 2023/249027

(57) **Abstract**

Provided is a quaternary ammonium salt having a specific structure, an organic structure-directing agent containing the quaternary ammonium salt, and a method for producing the quaternary ammonium salt. Also, provided is a CON-type zeolite, which is a mixed crystal in which a molar ratio (Si/Al) of silicon (Si) and aluminum (Al) is 200 or more and 2000 or less, and which contains 25 mass% or more of Polymorph A, a catalyst containing a CON-type zeolite, and a method for producing a lower olefin using the catalyst.

## Description

### Technical Field

The present invention relates to a novel quaternary ammonium salt. Also, it relates to an organic structure-directing agent that contains the quaternary ammonium salt, and a method for producing a zeolite using the organic structure-directing agent.

In addition, the present invention relates to an industrial method for producing a quaternary ammonium salt, and in particular, relates to a method for producing a quaternary ammonium salt containing a tricyclodecene ring and a tricyclodecane ring.

Further, the present invention relates to a CON-type zeolite, and more specifically relates to a CON-type zeolite which can achieve a long life when used as a catalyst, and a method for producing thereof. Furthermore, it relates to a catalyst containing the zeolite, and relates to a method for a lower olefin using the catalyst.

### Background Art

Quaternary ammonium salts are used in a wide range of fields, including as electrolytes for electrochemical elements such as electric double layer capacitors, batteries, and electrolytic capacitors, disinfectants, bactericides, preservatives, surfactants, fabric softeners, antistatic agents, phase transfer catalysts, dispersants for asphalt, cement, etc., anti-blocking agents for fertilizers and granular materials, anti-flocculating agents, and organic structure-directing agents in zeolite synthesis, and some have a variety of skeletons. Therefore, the quaternary ammonium having a novel structure is expected to be applicable to various fields due to their properties. In recent years, ammonium salts having polycyclic hydrocarbons have been used in the fields of medicine and resists due to their high hardness and high stability, and are also useful as organic structure-directing agents in zeolite synthesis.

Among these, quaternary ammonium salts having polycyclic hydrocarbons are expected to have various properties, and for example, N,N,N-trimethyl-8-ammonium tricyclo[5.2.1.0^{2,6}]decane hydroxide (Patent Literature 1) which is useful as an organic structure-directing agent for CON-type zeolite in zeolite synthesis, is known.

However, since quaternary ammonium salts having polycyclic hydrocarbons have complicated structures, it has been difficult to synthesize the basic skeleton thereof. The N,N,N-trimethyl-8-ammonium tricyclo[5.2.1.0^{2,6}]decane hydroxide described in Patent Literature 1 has been synthesized using 8-ketotricyclo[5.2.1.0^{2,6}]decane as a raw material, which is available as a reagent, and a method for synthesizing it on an industrial level has not been known.

Also, dicyclopentadiene is co-produced in large quantities during the production of ethylene by steam cracking of naphtha and heavy oil, and its main applications are as a raw material for resins such as polyester resins (Non-Patent Literature 1), as a raw material, etc., for organic synthesis of obtaining cyclopentadiene by thermal cracking, and as an ink additive, and therefore, a method for producing a quaternary ammonium salt having a polycyclic hydrocarbon structure using dicyclopentadiene as a raw material, such as tricyclo[5.2.1.0^{2,6}]decane ammonium salt, has not been known.

Further, zeolites also have properties such as a molecular sieve effect due to the pores derived from their skeleton structure, ion exchange capacity, catalytic capacity, and adsorption capacity, and are currently widely used as adsorbents, ion exchange agents, industrial catalysts, and environmental catalysts.

For example, CON-type zeolite is one of the zeolites that have pores of a 10-membered oxygen ring and two types of 12-membered oxygen rings, and has a topology classified as CON in the structure code defined by the International Zeolite Association. The CON-type zeolite is expected to be advantageous in terms of pore diffusion of reaction products compared to CHA-type zeolite, which only contains 8-membered ring structures, and MFI-type zeolite which only contains 10-membered ring structures.

As the CON-type zeolite, CIT-1, SSZ-26, and SSZ-33 are known. These zeolites have different composition ratios of Polymorph A and Polymorph B, which have different laminating patterns, and each is characterized by a different X-ray diffraction pattern. In order to synthesize these zeolites, it is known that ammonium salts each having a cyclic hydrocarbon structure are used as organic structure-directing agents. For example, N,N,N-trimethyl-cis-miltanyl ammonium hydroxide (Patent Literature 2) is known as an organic structure-directing agent for synthesizing CIT-1 type zeolite, hexamethyl[4.3.3.0]propellane-8,11-diammonium cation (Patent Literature 1) is known as an organic structure-directing agent for synthesizing SSZ-26 type zeolite, and N,N,N-trimethyl-8-ammonium tricyclo[5.2.1.0^{2,6}]decane hydroxide (Patent Literature 3) is known as an organic structure-directing agent for synthesizing SSZ-33 type zeolite.

In addition, CIT-1 zeolite is a CON-type zeolite whose main component is boron, and is a single crystal of Polymorph B. As a method for producing CIT-1 zeolite, for example, the Patent Literature 2 is known, and the CIT-1 zeolite (Si/B=25) which contains boron is obtained by hydrothermal synthesis at 175 °C for 7 days using N,N,N-trimethyl-cis-miltanyl ammonium hydroxide as an organic structure-directing agent. As an application of CIT-1 zeolite, the production of propylene (Patent Literature 4), and hydrocarbon trap (Non-Patent Literature 2), etc., are proposed. According to the Patent Literature 4, it is described that propylene can be efficiently produced by using methanol or/and dimethyl ether as a raw material, and using a catalyst which contains zeolite having CON-type structure (CIT-1 zeolite) as an active component. On the other hand, CIT-1 zeolite has a problem in that the organic structure-directing agent N,N,N-trimethyl-(-)-cis-miltanyl ammonium hydroxide, which is a raw material for synthesis, is expensive.

SSZ-26 zeolite is a CON-type zeolite whose main component is aluminum, and is a mixed crystal of Polymorph A and Polymorph B, which have different laminating patterns. According to the Non-Patent Literature 3, it is known that a composition ratio of Polymorph A and Polymorph B is 15:85. As a method for producing SSZ-26 zeolite, for example, Patent Literature 1 is known, aluminosilicates having Si/Al ratios of 12.5 to 17.5 is obtained by synthesis using hexamethyl[4.3.3.0]propellane-8,11-diammonium cation as an organic structure-directing agent.

SSZ-33 zeolite, as well as CIT-1 zeolite, is a CON-type zeolite whose main component is boron, and is a mixed crystal of Polymorph A and Polymorph B, which have different laminating patterns. According to Non-Patent Literature 3, it is known that a composition ratio of Polymorph A and Polymorph B in SSZ-33 zeolite is 30:70. As a method for producing SSZ-33 zeolite, for example, Patent Literature 3 is known, and it is described that a borosilicate having a Si/B of 15 to 25 can be synthesized by synthesizing at 160 °C for 6 to 10 days using N,N,N-trimethyl-8-ammonium tricyclo[5.2.1.0^{2,6}]decane hydroxide as an organic structure-directing agent. This Patent Literature 3 describes that as the T atoms, silicon, germanium, aluminum, gallium, iron, titanium, and vanadium are contained, and describes in the Examples that [B]-SSZ-33 zeolite, which contains boron together with silicon, is synthesized by hydrothermal synthesis of zeolite. It is described that the synthesis of aluminum-containing [Al]-SSZ-33 zeolite contains synthesizing [B]-SSZ-33 zeolite and then introducing aluminum by ion exchange with an aqueous aluminum nitrate solution.

The molar ratio (Si/Al) of silicon (Si) to aluminum (Al) in SSZ-33 zeolite is, for example, Si/Al = 30 in Non-Patent Literature 4, and basically, [Al]-SSZ-33 zeolite with a low Si/Al ratio is synthesized. The introduction of aluminum by the post method may result in incomplete insertion of Al into the zeolite skeleton, and tends to result in a zeolite with low stability of aluminum species. In order to solve this problem, there have been reported that [Al]-SSZ-33 zeolite was synthesized by a direct method, and in the Patent Literature 5, it discloses that SSZ-33 zeolite with Si/Al=5 to 100 can be directly synthesized by using 1,1'-(pentane-1,5-diyl)bis(3-methylcyclohexyl)piperidinium dication as an organic structure-directing agent. Regarding the particle diameter of SSZ-33 zeolite, for example, Non-Patent Literature 2 describes as 5 to 10 µm, and Non-Patent Literature 4 describes as 1 to 2 µm. In addition, as an application of SSZ-33 zeolite, such as naphtha reforming (Patent Literature 6) and hydrocarbon trapping (Non-Patent Literature 4) have been proposed .

### Citation List

### Patent Literature

Patent Literature 1: U.S. Patent No. 4,910,006
Patent Literature 2: U.S. Patent No. 5,512,267
Patent Literature 3: U.S. Patent No. 496,337
Patent Literature 4: JP 2013-245163 A
Patent Literature 5: U.S. Patent No. 8,647,601
Patent Literature 6: U.S. Patent No. 5,114,565

### Non Patent Literature

Non-Patent Literature 1: Journal of Synthetic Organic Chemistry, Japan, Vol.28, No.12 (1970) 1280-1284
Non-Patent Literature 2: Micro. Meso. Mater., 129,126-135 (2010)
Non-Patent Literature 3: J. Phys.Chem.,98,12040-12052 (1994)
Non-Patent Literature 4: J.Phys.Chem.B, 108, 13059-13061 (2004)

### Summary of Invention

### Technical Problem

The present invention is to provide a novel quaternary ammonium salt having a complex polycyclic hydrocarbon structure which can be applied to various applications. Also, it is to provide a novel organic structure-directing agent which is useful in zeolite synthesis, and to provide a method for producing CON-type zeolite.

Furthermore, as described above, it has been difficult to industrially produce a quaternary ammonium salts having polycyclic hydrocarbons. In addition, the use of dicyclopentadiene as a material for producing a quaternary ammonium salt has not been known.

The present invention has been made to solve the above problems, and an object of the present invention is to provide a method for industrially producing a quaternary ammonium salt having a polycyclic hydrocarbon structure.

Further, for the CON-type zeolite which is a mixed crystal of Polymorph A and Polymorph B, as represented by SSZ-33 zeolite and SSZ-26 zeolite, only aluminosilicates with low Si/Al ratio have been reported, as mentioned above. In addition, CON-type zeolite which is a mixed crystal of Polymorph A and Polymorph B, has not been studied as a catalyst for producing a lower olefin, and it has not been clarified what kind of catalyst shows a long life.

The present invention is to solve the above-mentioned problems, and an object of the present invention is to provide a CON-type zeolite that can be used as a catalyst in producing a lower olefin and can achieve a long life as a catalyst, a method for producing a CON-type zeolite, a catalyst containing a CON-type zeolite, and a lower olefin.

### Solution to Problem

The inventors have conducted study to solve the above problems, and have found that a quaternary ammonium salt having a specific structure is novel and a novel organic structure-directing agent useful in zeolite synthesis, and is useful in a method for producing CON-type zeolite, and completed the present invention.

In addition, since dicyclopentadiene is a compound which is co-produced in large quantities during the production of ethylene by steam cracking of naphtha and heavy oil, it accumulates year after year in petrochemical plants, and the amount of accumulation is increasing more than the amount of utilization, and it has been a compound which has problems for application development. Therefore, it is a cheap substance that can be obtained industrially in large quantities.

The inventors conducted extensive study in order to produce a quaternary ammonium salt having a tricyclo ring, which is a polycyclic hydrocarbon, and as a result, found that it can be produced industrially in high yield by using dicyclopentadiene as a raw material, and completed the present invention.

That is, the present invention provides a method for producing tricyclo[5.2.1.0^{2,6}]decene ammonium salt and tricyclo[5.2.1.0^{2,6}]decane ammonium salt, which can be manufactured industrially by using dicyclopentadiene as a raw material.

The present inventors also conducted study to solve the above problems and found that by containing silicon (Si) and aluminum (Al) as the constituent elements and by making the molar ratio (Si/Al) of silicon (Si) to aluminum (Al) appropriate, and by being a mixed crystal of Polymorph A and Polymorph B and making the content of Polymorph A appropriate, it is possible to provide a CON-type zeolite that can achieve a long service life as a catalyst, and completed the present invention.

A gist of the present invention is described in the following items [1] to [24].
[1] A quaternary ammonium salt represented by the following general formula (I) or the general formula (II). 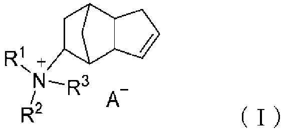
   (in which R¹, R², and R³ each independently represent an alkyl group, and A⁻ represents an anion), 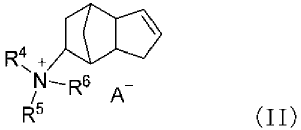
   (in which R⁴, R⁵, and R⁶ each independently represent an alkyl group, and A⁻ represents an anion).
[2] The quaternary ammonium salt according to the above item [1], in which the general formula (I) is represented by the following general formula (I-exo) and the general formula (II) is represented by the following (II-exo), 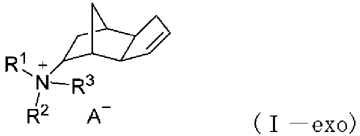
   (in which R¹, R², and R³ each independently represent an alkyl group, and A⁻ represents an anion), 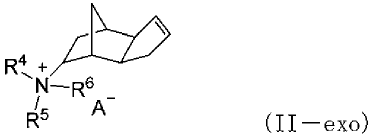
   (in which R⁴, R⁵, and R⁶ each independently represent an alkyl group, and A⁻ represents an anion).
[3] The quaternary ammonium salt according to the above item [1] or [2], in which R¹, R², R³, R⁴, R⁵ and R⁶ each independently represent an alkyl group having a carbon number of 1 to 6, and A is OH or a halogen atom.
[4] An organic structure-directing agent containing the quaternary ammonium salt according to any one of the above items [1] to [3].
[5] The organic structure-directing agent according to the above item [4] containing a quaternary ammonium salt represented by the following general formula (III) 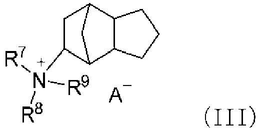 (in which R⁷, R⁸, and R⁹ each independently represent an alkyl group, and A⁻ represents an anion).
[6] The organic structure-directing agent according to the above item [5],
   in which the general formula (III) is a quaternary ammonium salt represented by the following general formula (III-exo), 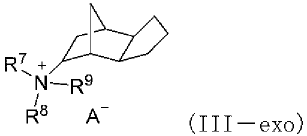 (in which R⁷, R⁸, and R⁹ each independently represent an alkyl group, and A⁻ represents an anion).
[7] A method for producing a quaternary ammonium salt represented by at least one of the following general formulas (I) and (II), or the general formula (III),
   in which dicyclopentadiene is used as a raw material, 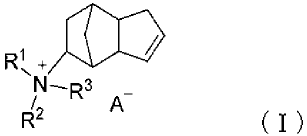
   in which (R¹, R², and R³ each independently represent an alkyl group, and A⁻ represents an anion), 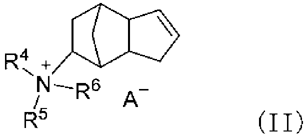
   (in which R⁴, R⁵, and R⁶ each independently represent an alkyl group, and A⁻ represents an anion). 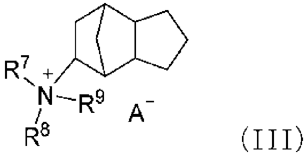
   (in which R⁷, R⁸, and R⁹ each independently represent an alkyl group, and A⁻ represents an anion).
[8] The method for producing a quaternary ammonium salt according to the above item [7], in which R¹, R², R³, R⁴, R⁵, R⁶, R⁷, R⁸ and R⁹ are each independently an alkyl group having a carbon number of 1 to 6, and A is each independently OH, HCO₃, MeCO₃, or a halogen atom.
[9] The method for producing a quaternary ammonium salt according to the above item [7] or [8], containing the following steps (1) to (4) in order,
   step (1): a step of producing 8-hydroxytricyclo[5.2.1.0^{2,6}]decene by hydrating dicyclopentadiene,
   step (2): a step of producing 8-ketotricyclo[5.2.1.0^{2,6}]decene by oxidizing the 8-hydroxytricyclo[5.2.1.0^{2,6}]decene obtained in the step (1),
   step (3): a step of producing dialkylaminotricyclo[5.2.1.0^{2,6}]decene by aminating the 8-ketotricyclo[5.2.1.0^{2,6}]decene obtained in the step (2),
   step (4): a step of producing trialkyltricyclo[5.2.1.0^{2,6}]decene ammonium salt by alkylating the dialkylaminotricyclo[5.2.1.0^{2,6}]decene obtained in the step (3).
[10] The method for producing a quaternary ammonium salt according to the above item [7] or [8], containing the following step (1), steps (2-2) to (4-2) in order,
   in which the step (2-2) and the step (3-2) are performed by using a solid catalyst,
   step (1): a step of producing 8-hydroxytricyclo[5.2.1.0^{2,6}]decene by hydrating dicyclopentadiene,
   step (2-2): a step of producing 8-ketotricyclo[5.2.1.0^{2,6}]decane by oxidizing and hydrogenating the 8-hydroxytricyclo[5.2.1.0^{2,6}]decene obtained in the step (1),
   step (3-2): a step of producing dialkylaminotricyclo[5.2.1.0^{2,6}]decane by aminating 8-ketotricyclo[5.2.1.0^{2,6}]decane obtained in the step (2-2),
   step (4-2): a step of producing trialkyltricyclo[5.2.1.0^{2,6}]decane ammonium salt by alkylating the dialkylaminotricyclo[5.2.1.0^{2,6}]decane obtained in the step (3-2).
[11] The method for producing a quaternary ammonium salt according to the above item [7] or [8], containing the following step (1), steps (2-2) to (4-2) in order,
   in which the step (2-2) and the step (3-2) are performed by using the same catalyst,
   step (1): a step of producing 8-hydroxytricyclo[5.2.1.0^{2,6}]decene by hydrating dicyclopentadiene,
   step (2-2): a step of producing 8-ketotricyclo[5.2.1.0^{2,6}]decane by oxidizing and hydrogenating the 8-hydroxytricyclo[5.2.1.0^{2,6}]decene obtained in the step (1),
   step (3-2): a step of producing dialkylaminotricyclo[5.2.1.0^{2,6}]decane by aminating 8-ketotricyclo[5.2.1.0^{2,6}]decane obtained in the step (2-2),
   step (4-2): a step of producing trialkyltricyclo[5.2.1.0^{2,6}]decane ammonium salt by alkylating the dialkylaminotricyclo[5.2.1.0^{2,6}]decane obtained in the step (3-2).
[12] The method for producing a quaternary ammonium salt according to any of the above items [9] to [11], containing the following step (A),
   step (A): a step of ion-exchanging the anion A⁻ of the trialkyltricyclo[5.2.1.0^{2,6}]decene ammonium salt obtained in the step (4) or the trialkyltricyclo[5.2.1.0^{2,6}]decane ammonium salt obtained in the step (4-2).
[13] The method for producing a quaternary ammonium salt according to the above item [12], in which the anion is exchanged with OH⁻ by the ion exchange in the step (A).
[14]A CON-type zeolite which satisfies the following (1) and (2),
   (1) containing silicon (Si) and aluminum (Al) as constituent elements, in which a molar ratio (Si/Al) of silicon (Si) and aluminum (Al) is 200 or more and 2000 or less,
   (2) which is a mixed crystal of Polymorph A and Polymorph B, and contains 25 mass% or more of Polymorph A.
[15] The CON-type zeolite according to the above item [14], containing silicon (Si) and boron (B) as constituent elements, in which a molar ratio (Si/B) of silicon (Si) and boron (B) is 5 or more and 100000 or less.
[16] The CON-type zeolite according to the above item [14] or [15], in which the zeolite is SSZ-33.
[17] The CON-type zeolite according to any of the above items [14] to [16], having an average primary particle diameter of 1000 nm or less.
[18] The CON-type zeolite according to any of the above items [14] to [17], in which a ratio (A2/A1) of the BET specific surface area (A2) calculated by BET plot to the external surface area (A1) calculated by t plot in a nitrogen adsorption/desorption measurement, is 10 or less.
[19] A method for producing a CON-type zeolite, which uses an organic structure-directing agent containing at least one of the quaternary ammonium salts represented by the following general formulas (I) and (II) as a raw material for zeolite synthesis, 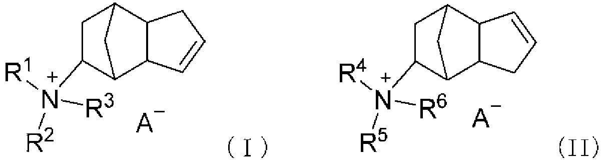 (in which R¹, R²,R³, R⁴, R⁵ and R⁶ each independently represent an alkyl group, and A⁻ represents an anion).
[20] The method for producing a CON-type zeolite according to the above item [19], in which R¹, R², R³, R⁴, R⁵ and R⁶ of the quaternary ammonium salt are all methyl groups, and A is OH.
[21] The method for producing a CON-type zeolite according to the above item [19], in which the organic structure-directing agent contains a quaternary ammonium salt represented by the following general formula (III), 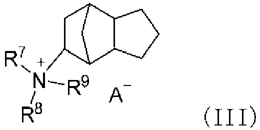 (in which R⁷, R⁸, and R⁹ each independently represent an alkyl group, and A⁻ represents an anion).
[22] A catalyst containing the CON-type zeolite according to any of the above items [14] to [18].
[23] The catalyst according to the above item [22], in which the catalyst is used in a reaction for producing a lower olefin.
[24] A method for producing a lower olefin, containing a step of contacting the catalyst according to the above item [23] with a raw material containing methanol or/and dimethyl ether.

### Advantageous Effects of Invention

According to the present invention, a novel quaternary ammonium salt can be provided. Furthermore, the quaternary ammonium salt is useful as an organic structure-directing agent for producing CON-type zeolite, and a method for producing CON-type zeolite can be provided.

Further, according to the present invention, a method for producing a quaternary ammonium salt that can be industrially produced using dicyclopentadiene as a raw material can be provided.

Furthermore, according to the present invention, it is possible to provide a CON-type zeolite that can achieve a long life as a catalyst. It is also possible to provide a method for producing CON-type zeolite that can achieve a long life as a catalyst. Furthermore, a catalyst containing the zeolite, and relates to a method for a lower olefin using the catalyst can be provided.

### Brief Description of Drawings

Fig. 1 is ¹³C-NMR spectrum of 8-ketotricyclo[5.2.1.0^{2,6}]decene.
Fig. 2 is a diagram showing the COSY spectrum of N,N,N-trimethyl-8-ammonium tricyclo[5.2.1.0^{2,6}]decane hydroxide (TCDEOH-III-exo).
Fig. 3 is a diagram showing the COSY spectrum of N,N,N-trimethyl-8-ammonium tricyclo[5.2.1.0^{2,6}]decene hydroxide (TCDEOH-1).
Fig. 4 is a diagram showing the HSQC spectrum of N,N,N-trimethyl-8-ammonium tricyclo[5.2.1.0^{2,6}]decane hydroxide (TCDEOH-III-exo).
Fig. 5 is a diagram showing the HMBC spectrum of N,N,N-trimethyl-8-ammonium tricyclo[5.2.1.0^{2,6}]decane hydroxide (TCDEOH-III-exo).
Fig. 6 is a diagram showing the NOESY spectrum of N,N,N-trimethyl-8-ammonium tricyclo[5.2.1.0^{2,6}]decane hydroxide (TCDEOH-III-exo).
Fig. 7 is a diagram showing the HSQC spectrum of N,N,N-trimethyl-8-ammonium tricyclo[5.2.1.0^{2,6}]decene hydroxide (TCDEOH-1).
Fig. 8 is a enlarged diagram showing the HSQC spectrum of N,N,N-trimethyl-8-ammonium tricyclo[5.2.1.0^{2,6}]decene hydroxide (TCDEOH-1).
Fig. 9 is a diagram showing the HMBC spectrum of N,N,N-trimethyl-8-ammonium tricyclo[5.2.1.0^{2,6}]decene hydroxide (TCDEOH-1).
Fig. 10 is a diagram showing the NOESY spectrum of N,N,N-trimethyl-8-ammonium tricyclo[5.2.1.0^{2,6}]decene hydroxide (TCDEOH-1).
Fig. 11 is ¹³C-NMR spectrum of N,N-dimethylaminotrycyclo[5.2.1.0^{2,6}]decene.
Fig. 12 is a diagram showing XRD patterns of the zeolites of Examples 3 and 4.
Fig. 13 is a diagram showing the results of powder X-ray diffraction of the zeolites (as-made type) according to Examples 8 to 10 and Comparative Examples 1 to 3.
Fig. 14 is a diagram showing the powder X-ray diffraction results of the zeolite (H type) of Example 9.
Fig. 15 is a scanning electron microscope (SEM) image of the zeolites according to Examples 8 to 10 and Comparative Examples 1 and 3.
Fig. 16 is a graph showing adsorption/desorption isotherms of the zeolites according to Examples 8 to 10 and Comparative Examples 1 to 3. For ease of viewing the graphs, the vertical axes for Examples 1 and 2 and Comparative Examples 1 and 2 are made to be +400, +300, +200, and +100 [ml (STP)/g], respectively. The adsorption side is represented by a filled marker, and the desorption side is represented by an unfilled marker.
Fig. 17 is a graph showing the transition of methanol conversion in the production of a lower olefin using the zeolites of Examples 8 to 10 and Comparative Examples 1 to 3 as catalysts.

### Description of Embodiments

The present invention will be described in detail below, but an explanation of the constituent elements described below is an example (representative example) of an embodiment of the present invention, and the present invention is not limited to these contents, and various modifications can be made within the scope of the gist.

### <First Embodiment>

### [Quaternary ammonium salt]

A quaternary ammonium salt of the present invention is represented by the following general formula (I) or general formula (II). 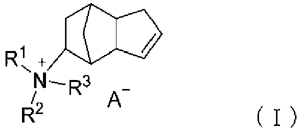
(in which R¹, R², and R³ each independently represent an alkyl group, and A⁻ represents an anion), 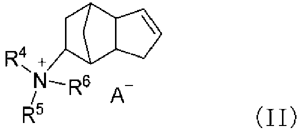
(in which R⁴, R⁵, and R⁶ each independently represent an alkyl group, and A⁻ represents an anion).

In general formulas (I) and (II), the substituents R¹ to R⁶ are alkyl groups and may be the same or different, and the specific examples include linear, branched and cyclic alkyl groups having a carbon number of 1 to 6, such as a methyl group, an ethyl group, an n-propyl group, an isopropyl group, an n-butyl group, a 2-methylpropyl group, an n-pentyl group, a 2-methylbutyl group, a 2,2-dimethylpropyl group, an n-hexyl group, a 2-methylpentyl group, a 3-methylpentyl group, a 2-ethylbutyl group, a 2,3-dimethylbutyl group, a cyclopropyl group, a cyclobutyl group, a cyclopentyl group, a 2-methylcyclopentyl group, a 3-methylcyclopentyl group and a cyclohexyl group, and among these, an alkyl groups having a carbon number of 1 to 3, such as a methyl group, an ethyl group, an n-propyl group and an isopropyl group are preferred, and a methyl group and an ethyl group are further preferred.

In the general formulas (I) and (II), A in the anion portion A⁻ is not limited, and is preferably OH or a halogen atom.

Among these, in the general formulas (I) and (II), it is preferred that the substituents R¹ to R⁶ are each independently an alkyl group having a carbon number of 1 to 6, and A is OH or a halogen atom.

The ring structure in the structure of general formulas (I) and (II) may be either exo type or endo type, however when used as an organic structure-directing agent, the more preferred structures are the exo types represented by the following general formulas (I-exo) and (II-exo). 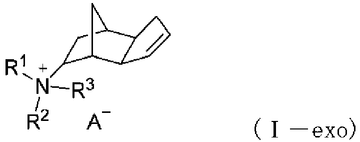
(in which R¹, R², and R³ each independently represent an alkyl group, and A⁻ represents an anion), 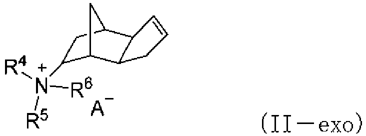
(in which R⁴, R⁵, and R⁶ each independently represent an alkyl group, and A⁻ represents an anion).

### [Organic structure-directing agent]

An organic structure-directing agent of the present invention contains the quaternary ammonium salt. The organic structure-directing agent may be used alone or in combination of two or more types. When two or more types of organic structure-directing agents are combined, a quaternary ammonium salt represented by general formula (I) may be combined with a quaternary ammonium salt represented by general formula (II), or quaternary ammonium salts represented by general formula (I) may be combined with each other, or quaternary ammonium salts represented by general formula (II) may be combined with each other.

Another preferred embodiment of the organic structure-directing agent is to contain a quaternary ammonium salt represented by the following general formula (III) having a similar skeleton. Therefore, there is no problem even when the compound represented by general formula (III) is mixed as a by-product during production. 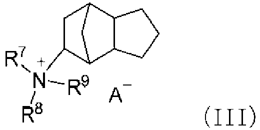 (in which R⁷, R⁸, and R⁹ each independently represent an alkyl group, and A⁻ represents an anion).

In general formula (III), the substituents R⁷ to R⁹ are alkyl groups and may be the same or different, and the specific examples include linear, branched and cyclic alkyl groups having a carbon number of 1 to 6, such as a methyl group, an ethyl group, an n-propyl group, an isopropyl group, an n-butyl group, a 2-methylpropyl group, an n-pentyl group, a 2-methylbutyl group, a 2,2-dimethylpropyl group, an n-hexyl group, a 2-methylpentyl group, a 3-methylpentyl group, a 2-ethylbutyl group, a 2,3-dimethylbutyl group, a cyclopropyl group, a cyclobutyl group, a cyclopentyl group, a 2-methylcyclopentyl group, a 3-methylcyclopentyl group and a cyclohexyl group, and an alkyl groups having a carbon number of 1 to 3, such as a methyl group, an ethyl group, an n-propyl group and an isopropyl group are preferred, and a methyl group and an ethyl group are further preferred.

A in the anion portion A⁻ of the quaternary ammonium salt is not limited, and is preferably OH or a halogen atom.

The ring structure in the structure of general formula (III) may be either exo type or endo type, however when used as an organic structure-directing agent, the more preferred structures are the exo types represented by the following general formula (III-exo). 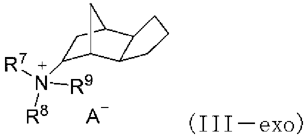 (in which R⁷, R⁸, and R⁹ each independently represent an alkyl group, and A⁻ represents an anion).

### [Method for producing quaternary ammonium salt]

Next, a method for producing a quaternary ammonium salt of the present invention will be described.

A method for producing a quaternary ammonium salt of the present invention is not limited, however as an example, a method for producing from a ketone compound shown below will be shown.

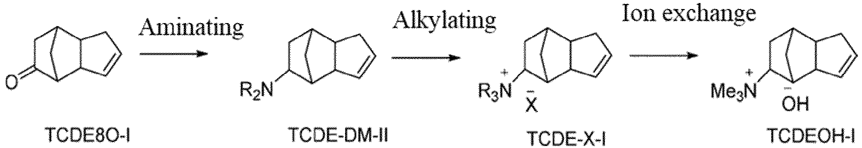

As a method for producing an amino compound (TCDE-DM-II) from a ketone compound (TCDE8O-I), a reductive amination method using a dialkylamine or a method of alkylating after introducing an amino group using NH₃+H₂ can be applied. Furthermore, by alkylating using an alkyl halide or dialkyl carbonate, a quaternary ammonium salt (TCDE-XI) can be easily obtained from a tertiary amine (TCDE-DM-II). Thereafter, when the anionic portion is to be converted to OH⁻, it can be easily converted by ion exchange methods using ion exchange resins or salts having the necessary anions, and can produce an ammonium OH salt (TCDEOH-I).

### <<Second Embodiment>>

### [Method for producing quaternary ammonium salt]

A method for producing a quaternary ammonium salt of the present invention is a method for producing a quaternary ammonium salt represented by at least one of the following general formulas (I) and (II), or the general formula (III), in which dicyclopentadiene is used as a raw material. 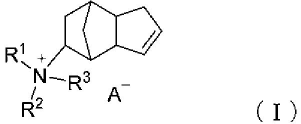
(in which R¹, R², and R³ each independently represent an alkyl group, and A⁻ represents an anion), 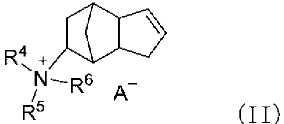
(in which R⁴, R⁵, and R⁶ each independently represent an alkyl group, and A⁻ represents an anion). 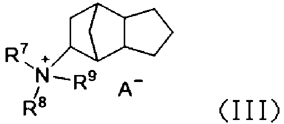
(in which R⁷, R⁸, and R⁹ each independently represent an alkyl group, and A⁻ represents an anion).

In general formulas (I) to (III), the substituents R¹ to R⁹ are alkyl groups and may be the same or different, and the specific examples include linear, branched and cyclic alkyl groups having a carbon number of 1 to 6, such as a methyl group, an ethyl group, an n-propyl group, an isopropyl group, an n-butyl group, a 2-methylpropyl group, an n-pentyl group, a 2-methylbutyl group, a 2,2-dimethylpropyl group, an n-hexyl group, a 2-methylpentyl group, a 3-methylpentyl group, a 2-ethylbutyl group, a 2,3-dimethylbutyl group, a cyclopropyl group, a cyclobutyl group, a cyclopentyl group, a 2-methylcyclopentyl group, a 3-methylcyclopentyl group and a cyclohexyl group, and among these, an alkyl groups having a carbon number of 1 to 3, such as a methyl group, an ethyl group, an n-propyl group and an isopropyl group are preferred, and a methyl group and an ethyl group are further preferred.

In the general formulas (I) to (III), A in the anion portion A⁻ is not limited, however, preferably, each independently is OH, HCO₃, MeCO₃, or a halogen atom.

Among these, in the general formulas (I) to (III), it is preferred that the substituents R¹ to R⁹ are each independently an alkyl group having a carbon number of 1 to 6, and A is each independently OH or a halogen atom.

Dicyclopentadiene used as a raw material in the method for producing a quaternary ammonium salt of the present invention is a compound that is co-produced in large quantities during the production of ethylene by steam cracking of naphtha and heavy oil, and is an inexpensive substance that is available in large quantities industrially. Therefore, by using dicyclopentadiene as a raw material, raw material costs can be kept low, and a quaternary ammonium salt can be produced inexpensively.

As the method for producing a quaternary ammonium salt of the present invention, there is no particular limitation as to any process, as long as it is a method for producing a quaternary ammonium salt represented by at least one of general formulas (I) and (II), or general formula (III), using dicyclopentadiene (hereinafter referred to as "DCPD") as a raw material.

As an example of the method for producing the quaternary ammonium salt of the present invention, for example, the following step can be shown. It is a method containing the steps (1) to (4) in order, to produce a quaternary ammonium salt (tricyclo[5.2.1.0^{2,6}]decene ammonium salt) represented by the general formulas (I) and (II). Alternatively, it is a method containing the steps (1) and the steps (2-2) to (4-2) in order, to produce a quaternary ammonium salt represented by general formula (III) (tricyclo[5.2.1.0^{2,6}]decane ammonium salt).

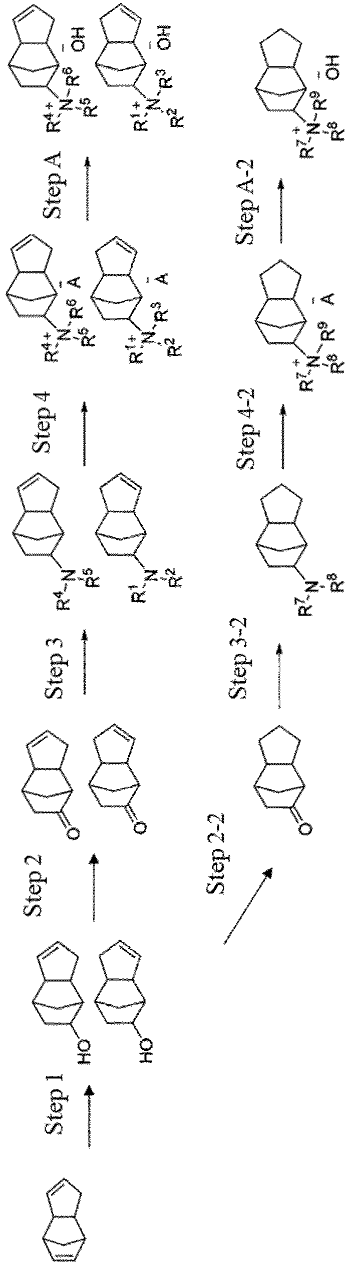

Each step of the method for producing the quaternary ammonium salts represented by the general formulas (I) and (II) will be described below. The general formulas (I) and (II) can be produced via steps (1) to (4).

The step (1) is a step of producing 8-hydroxytricyclo[5.2.1.0^{2,6}]decene by hydrating dicyclopentadiene. That is, this is a step of hydrating DCPD and introducing OH group. A method for introducing OH group is not particularly limited, as long as it is a method in which the olefin is substituted with a OH group, and the product may be produced as a mixture of isomers. A method of hydration using an acid such as sulfuric acid or acetic acid is generally capable of introducing OH group at low cost and in high yield. Alternatively, an acidic solid catalyst can be used to introduce OH group. Specific examples include acid type ion exchange resins and acid type metal-containing catalysts. In this case, the reaction proceeds regardless of whether a solvent is used or not, therefore it may be used differently depending on the reaction conditions and substituents. The OH body obtained in the step (1) can be purified by a conventional purification method used in organic synthesis, such as distillation or short column purification, and is preferably isolated as a colorless and transparent liquid by distillation purification.

The step (2) is a step of producing 8-ketotricyclo[5.2.1.0^{2,6}]decene by oxidizing the 8-hydroxytricyclo[5.2.1.0^{2,6}]decene obtained in the above step (1). That is, this is a step of converting the OH body obtained in the step (1) into a ketone body. The step (2) is not particularly limited as long as it is a method for converting an OH body into a ketone body, and is preferably performed using a solid catalyst. As the solid catalyst, a Cu-based catalyst, a Ni-based catalyst, a Co-based catalyst, a Cr-based catalyst, an Fe-based catalyst, a Ru-based catalyst, or a Pd-based catalyst can be used as a single catalyst or a composite catalyst, however, in order to selectively produce an unsaturated ketone body, it is suitable to use a catalyst with weak hydrogenation ability, and a Cu-based catalyst, a Cr-based catalyst, or a composite catalyst containing these metals is preferably used, and a composite catalyst of copper and chromium is most preferred. These solid catalysts can be removed by filtration after the reaction, allowing the product to be isolated without complex post-processing steps.

The step (3) is a step of producing dialkylaminotricyclo[5.2.1.0^{2,6}]decene by aminating the 8-ketotricyclo[5.2.1.0^{2,6}]decene obtained in the above step (2). That is, this is a step of introducing an amino group into the ketone body obtained in the step (2). The introduction of an amino group in the step (3) is not limited as long as it is a method capable of introducing an amino group in an amination reaction, however it is preferable to use a reductive amination reaction using a solid catalyst. In the reductive amination reaction, hydrogen, formic acid, borohydrides, ammonium formate, etc., can be used as a reducing agent. When hydrogen is used as the reducing agent, there is no limitation on the hydrogen pressure, however it is preferable to charge hydrogen in a pressure-resistant vessel and carry out the reaction under slight pressure to high pressure. **In** the case where there is a shortage of reducing agent during the reaction under slight pressure, it is possible to carry out hydrogen supplement. There are no limitations on the amino group-introducing agent as long as it is capable of aminating, however generally, dialkylamines, dialkylformamides, etc., can be used. Some dialkylamines have low boiling points, but can be used as a liquefied gas or in a state dissolved in a solvent. From the viewpoint of economy and simplification of post-processing processes, a method using a solid catalyst in combination with hydrogen is desirable. **In** this step, similar to the step (2), the solid catalyst to be used can be removed by filtration after the reaction, allowing the product to be isolated without complex post-processing steps.

The step (4) is a step of producing trialkyltricyclo[5.2.1.0^{2,6}]decene ammonium salt by alkylating the dialkylaminotricyclo[5.2.1.0^{2,6}]decene obtained in the above step (3). That is, this is a step of introducing an alkyl group into the amino group obtained in the step (3). The introduction of an alkyl group in the step (4) is not limited as long as the method is capable of introducing an alkyl group and producing a quaternary ammonium salt in the alkylation reaction. A method using a halogenated alkyl or a method using a dialkyl carbonate can be easily performed. **In** the method using a halogenated alkyl, it is possible to synthesis by being dissolved in a solvent in which the substrate is soluble, and by stirring at a temperature between 0 °C to the reflux temperature of the solvent using an equivalent amount or more of a halogenated alkyl. In the method using a dialkyl carbonate, only by charging a substrate, an equal or greater amount of dialkyl carbonate to the substrate, and a solvent into an autoclave, and by heating and stirring under slight pressure, a quaternary ammonium salt can be produced. It is possible without using a solvent, however a solvent in which the substrate is soluble can be used, and an alcohol solvent such as methanol, ethanol, or propanol, or a mixed solvent in which a small amount of H₂O is added to these alcohol solvents, are preferred. The reaction temperature is not particularly limited as long as the reaction proceeds, and is preferably 100°C to 250°C, further preferably 150°C to 230°C.

The quaternary ammonium salt produced in step (4) can be further converted to a preferred anion in step (A). The step (A) is a step of ion-exchanging the anion A⁻ of the trialkyltricyclo[5.2.1.0^{2,6}]decene ammonium salt obtained in the above step (4). The conversion method is not particularly limited, however the conversion can be easily performed by ion exchange using an ion exchange resin or a salt having the required anion. A in the preferred anion A⁻ is OH, HCO₃, MeCO₃ or a halogen atom, and is particularly preferably converted to OH.

Next, a method for producing the saturated quaternary ammonium compound represented by the general formula (III) will be described. The general formulas (III) can be produced via step (1) and the steps (2-2) to (4-2).

The step (1) is a step of producing 8-hydroxytricyclo[5.2.1.0^{2,6}]decene by hydrating dicyclopentadiene. Since the step (1) can be carried out in the same manner as the step (1) of the general formula (I) and/or (II), the explanation thereof will be omitted.

The step (2-2) is a step of producing 8-ketotricyclo[5.2.1.0^{2,6}]decane by oxidizing and hydrogenating the 8-hydroxytricyclo[5.2.1.0^{2,6}]decene obtained in the above step (1). That is, this is a step of converting the OH body obtained in step (1) into a saturated ketone body. The step (2-2) is not particularly limited as long as it is a method of converting an OH body into a saturated ketone body using a solid catalyst. As the solid catalyst, a Cu-based catalyst, a Ni-based catalyst, a Co-based catalyst, a Cr-based catalyst, an Fe-based catalyst, a Ru-based catalyst, or a Pd-based catalyst can be used as a single catalyst or a composite catalyst, however, in order to selectively produce saturated ketone bodies, it is suitable to use a catalyst with strong hydrogenation ability, and a Ni-based catalyst, a Ru-based catalyst, a Pd-based catalyst, or a composite catalyst containing these metals can preferably be used.

The step (3-2) is a step of producing dialkylaminotricyclo[5.2.1.0^{2,6}]decane by aminating 8-ketotricyclo[5.2.1.0^{2,6}]decane obtained in the above step (2-2). That is, this is a step of introducing an amino group into the ketone body obtained in the step (2-2). Since the introduction of amino group of the step (3-2) can be carried out in the same manner as the step (3), the explanation thereof will be omitted.

The step (4-2) is a step of producing trialkyltricyclo[5.2.1.0^{2,6}]decane ammonium salt by alkylating the dialkylaminotricyclo[5.2.1.0^{2,6}]decane obtained in the above step (3-2). That is, this is a step of introducing an alkyl group into the amino group obtained in the step (3-2). Since the introduction of alkyl group of the step (4-2) can be carried out in the same manner as the step (4), the explanation thereof will be omitted.

The quaternary ammonium salt produced in step (4-2) can be further converted to a preferred anion in step (A). The step (A) is a step of ion-exchanging the anion A⁻ of the trialkyltricyclo[5.2.1.0^{2,6}]decane ammonium salt obtained in the above step (4-2). Since the method for converting to a preferred anion in step (A) can be carried out in the same manner as the method for converting the anion of the trialkyltricyclo[5.2.1.0^{2,6}]decene ammonium salt in step (A) above, the explanation thereof will be omitted.

Furthermore, steps (2) to (3) or steps (2-2) to (3-2) can be performed using the same catalyst, respectively. In that case, the reactions can be carried out sequentially in the same reactor.

### <<Third Embodiment>>

### [CON-type zeolite]

One embodiment of the present invention is a CON-type zeolite which is a mixed crystal of Polymorph A and Polymorph B, and contains 25 mass% or more of Polymorph A.

By making the mass ratio of Polymorph A equal to or greater than the above lower limit, the catalyst life can be improved. Specifically, Polymorph A may be contained in amount of 25 mass% or more, and the mass ratio of Polymorph A may be 90% or less, preferably 60% or less, further preferably 40% or less, and most preferably 35% or less. This range is a range of the CON-type zeolites commonly referred to as SSZ-33. That is, a mixed crystal of Polymorph A and B, with Polymorph A in a range of 25 mass% or more and 35 mass% or less, is most preferred. The SSZ-33 zeolite of as-made type and the SSZ-33 zeolite of H type are characterized by their X-ray diffraction patterns as shown in Tables 1 and 2, respectively. However, when measuring an actually synthesized zeolite, it is influenced by the growth direction of zeolite, the ratio of constituent elements, adsorbed substances, the presence of defects, drying conditions, etc., therefore, there is a slight shift in the intensity ratio and peak position of each peak, and a tolerance of about 20% for the intensity ratio and about 5% for the peak position is allowable. As described in U.S. Patent No. 5512267, CIT-1 zeolite, which is a single crystal of Polymorph B, has peaks specific to CIT-1 zeolite at around 2θ = 9.0, 15.5, and 24.5°, respectively, therefore can be distinguished from SSZ-33 zeolite, which contains approximately 30 mass% of Polymorph A. In addition, also for SSZ-26 zeolite containing 15 mass% of Polymorph A, it can be distinguished since a specific peak is observed near 2θ=9.0° as described in J. Phys. Chem., 98, 12040-12052 (1994). As described in J. Phys. Chem., 98, 12040-12052 (1994), the mass ratio of Polymorph A can be identified by comparing the X-ray diffraction pattern with the simulation results in which the mixing ratio of Polymorph A and Polymorph B is varied, and by the presence or absence of a peak specific to that ratio, as well as the peak position and intensity.

**Table 1**

| 2θ (°) | d (Å) | Relative Intensity |
|---|---|---|
| 7.97 | 11.09 | Very strong |
| 14.37 | 6.16 | Medium |
| 15.89 | 5.58 | Medium |
| 16.88 | 5.25 | Weak |
| 20.00 | 4.44 | Medium |
| 20.60 | 4.31 | Strong |
| 21.61 | 4.11 | Medium |
| 22.16 | 4.01 | Strong |
| 23.17 | 3.84 | Strong |
| 23.29 | 3.82 | Very strong |
| 25.53 | 3.49 | Medium |
| 26.97 | 3.31 | Medium |
| 28.84 | 3.10 | Weak |
| 29.30 | 3.05 | Weak |

**Table 2**

| 2θ (°) | d (Å) | Relative Intensity |
|---|---|---|
| 7.95 | 11.12 | Very strong |
| 8.40 | 10.53 | Medium |
| 13.41 | 6.60 | Weak |
| 14.36 | 6.17 | Weak |
| 16.88 | 5.25 | Weak |
| 20.53 | 4.33 | Medium |
| 21.59 | 4.12 | Weak |
| 22.18 | 4.01 | Medium |
| 23.15 | 3.84 | Medium |
| 23.27 | 3.82 | Medium |
| 25.49 | 3.49 | Weak |
| 26.85 | 3.32 | Weak |
| 28.84 | 3.10 | Weak |
| 29.26 | 3.05 | Weak |

The radiation used in the measurement was CuKα, and the relationship between 2θ and the interplanar spacing d was calculated. In addition, the relative intensity was defined as 100 for the strongest peak, with peaks greater than 60 and less than 100 being "very strong", peaks greater than 30 and less than 60 being "strong", peaks greater than 10 and less than 30 being "medium", and peaks greater than 0 and less than 10 being "weak".

In this embodiment, a CON-type zeolite (CON-type zeolite of this embodiment which is a mixed crystal of Polymorph A and Polymorph B, and contains 25 mass% or more of Polymorph A, and hereinafter, simply referred to as CON-type zeolite) contains silicon (Si) and aluminum (Al) as constituent elements, in which a molar ratio (Si/Al) of silicon (Si) and aluminum (Al) is 200 or more and 2000 or less.

When the molar ratio (Si/Al) of silicon (Si) to aluminum (Al) is equal to or more than the above lower limit, the catalyst life can be improved. Specifically, the molar ratio (Si/Al) of silicon (Si) to aluminum (Al) may be 200 or more, preferably 250 or more, more preferably 275 or more, further preferably 300 or more, and particularly preferably 325 or more. Furthermore, by having the molar ratio (Si/Al) of silicon (Si) to aluminum (Al) of equal to or less than the upper limit, sufficient activity as a catalyst can be obtained. The upper limit of the molar ratio (Si/Al) of silicon (Si) to aluminum (Al) may be 2000 or less, preferably 1500 or less, more preferably 1000 or less, further preferably 750 or less, and particularly preferably 500 or less.

Setting the molar ratio (Si/Al) of silicon (Si) to aluminum (Al) to a specific range, for example, can be achieved by adding aluminum to a zeolite synthesis raw material gel in the production process of CON-type zeolite and appropriately adjusting the synthesis conditions.

The elements other than silicon (Si) and aluminum (Al) contained as constituent elements in the CON-type zeolite of this embodiment are not particularly limited, however, examples thereof include one kind or two kinds or more selected from boron (B), titanium (Ti), vanadium (V), iron (Fe), zinc (Zn), gallium (Ga), germanium (Ge), zirconium (Zr), and tin (Sn).

Specifically, preferred examples include crystalline aluminosilicates containing Si and Al as constituent elements, as well as crystalline galloaluminosilicates containing Ga. These zeolites have excellent catalytic activity since Al and Ga in the zeolite skeleton become acid sites and act as active sites for catalytic reactions.

In addition, since the CON-type zeolite can be easily obtained by adding a boron compound to the raw material gel described below, the CON-type zeolite may contain B (boron) as a constituent element. Examples of such materials include crystalline boroaluminosilicate containing Si, Al and B as constituent elements, and crystalline galloboroaluminosilicate further containing Ga.

In the case of a crystalline galloaluminosilicate or crystalline galloboroaluminosilicate containing Ga in addition to Si and Al as constituent elements, a Ga content ratio is not particularly limited, however it is preferable that a Si/Ga molar ratio is 100 or more, particularly 200 or more, especially 400 or more, and 8000 or less, particularly 4000 or less, especially 2000 or less, and a Ga/Al molar ratio is 0.05 or more, particularly 0.1 or more, especially 0.2 or more, and 4 or less, particularly 2 or less, especially 1 or less, in order to obtain a zeolite catalyst having sufficient catalytic activity and extremely excellent catalytic life.

In addition, when the CON-type zeolite of this embodiment has B (boron), a Si/B molar ratio is not particularly limited, and is usually 3 or more, preferably 5 or more, more preferably 10 or more, and further preferably 20 or more, and the upper limit is preferably 100,000 or less, more preferably 50,000 or less, and further preferably 10,000 or less.

The contents of Si, Al, Ga, B, etc., in the CON-type zeolite of the present embodiment are usually values measured for the produced CON-type zeolite by inductively coupled plasma atomic emission spectrometry (ICP-AES), etc., and are not the ratios of the raw materials charged.

The average primary particle diameter of the CON-type zeolite of this embodiment is preferably 1000 nm or less. When the average primary particle diameter is equal to or less than the upper limit, a zeolite catalyst having an extremely excellent catalytic life can be obtained. The average primary particle diameter is more preferably 800 nm or less, further preferably 700 nm or less, and particularly preferably 600 nm or less. The lower limit is not particularly limited, however it is usually 20 nm or more, preferably 40 nm or more, more preferably 60 nm or more.

Both the "primary particle diameter" and the "average primary particle diameter" can be calculated using a scanning electron microscope (SEM).

Here, the "primary particle" means the smallest particle in which no grain boundary can be identified. In the present invention, SEM images of zeolite catalysts are acquired, and the smallest particle that corresponds to the zeolite in the SEM image and for which no grain boundaries are identified is determined as the "primary particle. In the present invention, the primary particles do not necessarily exist as single particles, and may form secondary particles by aggregation, etc. Even when secondary particles are formed, primary particles on the surfaces of the secondary particles can be distinguished in the SEM image.

The "average primary particle diameter" is measured as follows. That is, 50 primary particles contained in the SEM image of the zeolite catalyst are randomly selected, the major axis (the length of the longest straight line when connecting one end of the primary particle with the other end of the primary particle) is measured for each of the selected 50 primary particles, and the arithmetic average value of the major axes measured for the 50 particles is determined as the "average primary particle diameter". However, when the entire zeolite catalyst contains less than 50 primary particles, the major axis of each of all primary particles contained in the zeolite catalyst is measured, and the average value thereof is determined as the "average primary particle diameter".

Setting the average primary particle diameter within a specific range, for example, can be achieved by using CON-type zeolite having a small particle size as seed crystals, or by adding a surfactant to a raw material gel for synthesizing zeolite in the production process of CON-type zeolite, as described below.

For the CON-type zeolite of this embodiment, a ratio (A2/A1) of the BET specific surface area (A2) calculated by BET plot to the external surface area (A1) calculated by t plot is preferably 10 or less. When (A2/A1) is equal to or less than the upper limit, a zeolite catalyst having an extremely excellent catalytic life can be obtained. (A2/A1) is more preferably 9.5 or less, further preferably 9.0 or less, and further more preferably 8.5 or less, and particularly preferably 8.0 or less. The lower limit is not particularly limited, and is usually 2.0 or more.

The ratio (A2/A1) of the BET specific surface area (A2) to the external surface area (A1), as well as the BET specific surface area (A2) and the micropore volume (A3) can be calculated from nitrogen adsorption/desorption measurements, which can be performed by using, for example, a Belsorp-mini II manufactured by MicrotracBEL Corp. In addition, the data analysis can be performed using analysis software BELMaster manufactured by MicrotracBEL Corp. Here, the BET specific surface area (A2) is calculated by BET plotting of the measurement data with a relative pressures (P/P₀) of 0.002 to 0.06. The external surface area (A1) and the micropore volume (A3) are calculated by t-plotting of the data with a relative pressures (P/P₀) of 0.20 to 0.42. Note that Harkins-Jura is used as a standard isotherm.

Setting the (A2/A1) within a specific range, for example, can be achieved by using CON-type zeolite having a small particle size as seed crystals, or by adding a surfactant to a raw material gel for synthesizing zeolite in the production process of CON-type zeolite, as described below.

The BET specific surface area (A2) of the CON-type zeolite of this embodiment is not particularly limited, and is preferably 400 m²/g or more, more preferably 500 m²/g or more, further preferably 550 m²/g or more, further more preferably 570 m²/g or more, and particularly preferably 590 m²/g or more. The upper limit of the BET specific surface area (A2) is not particularly limited, and is usually 2000 m²/g or less.

The micropore volume (A3) of the CON-type zeolite of this embodiment is not particularly limited, and is usually 0.1 ml/g or more, preferably 0.15 ml/g or more, more preferably 0.18 ml/g or more, and is usually 3 ml/g or less, preferably 2 ml/g or less.

The ion exchange sites of the CON-type zeolite of this embodiment are not particularly limited, and may be H type or may be exchanged with metal ions. Here, examples metal ions specifically include alkali metal ions, alkaline earth metal ions, cerium, tungsten, manganese, iron.

### <Method for Producing CON-type Zeolite>

Hereinafter, a method for producing the CON-type zeolite of this embodiment will be described.

A CON-type zeolite can generally be prepared by a hydrothermal synthesis method. For example, an alkali source, an organic structure-directing agent, etc. are added to water and stirred, and an aluminum source, a gallium source, a boron source, a silica source, etc., are further added to generate a uniform gel, and the obtained raw material gel is maintained at 120 to 240 °C in a pressurized and heated vessel such as an autoclave to crystallize it. In this embodiment, it is preferable to produce CON-type zeolite by a direct method in which an aluminum source is mixed with a raw material gel. It is believed that by synthesizing by the direct method, insertion of aluminum into the zeolite skeleton becomes easier to occur, and becomes easier to be a zeolite with high stability of aluminum species.

Further, during crystallization, seed crystals may be added as necessary, and in terms of manufacturability, the addition of seed crystals is preferred from the viewpoint that it can be synthesized efficiently. As the seed crystals, it is preferable to use CON-type zeolite or BEA-type zeolite, and it is particularly preferable to use CON-type zeolite.

Furthermore, during the crystallization, a surfactant may be added to the raw material gel. By adding a surfactant, the zeolite becomes fine particles, and it becomes easier to adjust the average primary particle diameter and the value of (A2/A1) to the desired range.

As the organic structure-directing agent, an organic structure-directing agent containing a quaternary ammonium salt represented by the following general formula (I) or (II) is preferred, and it can be used alone or in combination. In particular, it is more preferable to use quaternary ammonium salts represented by the following general formulas (IV) and (V). In addition, a quaternary ammonium salt represented by the following general formula (III) can be used. The ring structure in the structure of general formulas (I), (II) and (III) may be either exo type or endo type, and when used as an organic structure-directing agent, the more preferred structures are the exo types represented by the following general formulas (I-exo), (II-exo), and (III-exo).

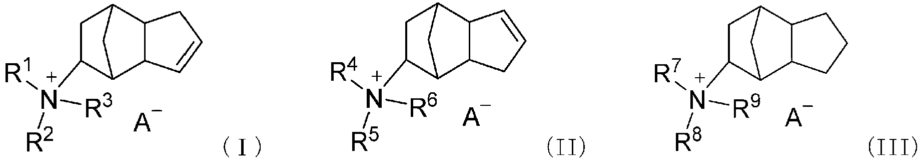

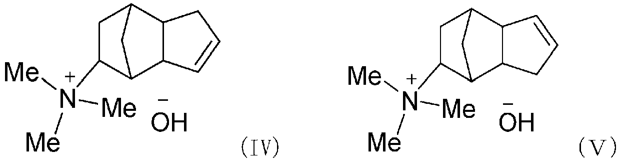

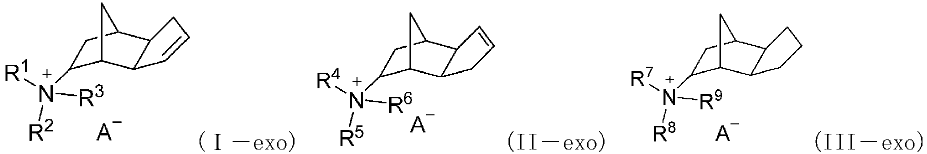

(R¹ to R⁹ represent an alkyl group, and A⁻ represents an anion. Examples of alkyl group include linear, branched and cyclic alkyl groups having a carbon number of 1 to 6, such as a methyl group, an ethyl group, an n-propyl group, an isopropyl group, an n-butyl group, a 2-methylpropyl group, an n-pentyl group, a 2-methylbutyl group, a 2,2-dimethylpropyl group, an n-hexyl group, a 2-methylpentyl group, a 3-methylpentyl group, a 2-ethylbutyl group, a 2,3-dimethylbutyl group, a cyclopropyl group, a cyclobutyl group, a cyclopentyl group, a 2-methylcyclopentyl group, a 3-methylcyclopentyl group and a cyclohexyl group, and among these, an alkyl groups having a carbon number of 1 to 3, such as a methyl group, an ethyl group, an n-propyl group and an isopropyl group, are preferred, and a methyl group and an ethyl group are more preferred, and a methyl group is particularly preferred. The anion is preferably OH⁻ or a halogen anion, and more preferably OH⁻.

As the surfactant, a cationic surfactant, an anionic surfactant, a nonionic surfactant, an amphoteric surfactant, etc., can be used alone or in combination. As the surfactant, a cationic surfactant that acts as an appropriate crystal growth inhibitor is preferred, and examples thereof include hexadecyltrimethylammonium bromide, hexadecyltrimethylammonium chloride, and hexadecyltrimethylammonium hydroxide.

The amount of surfactant added to the raw material gel is not particularly limited, however the molar ratio of the surfactant to Si in the raw gel is preferably 0.001 or more, further preferably 0.002 or more, particularly preferably 0.005 or more, and is preferably 0.05 or less, further preferably 0.03 or less, particularly preferably 0.015 or less.

After the raw material gel is crystallized, the crystallized raw material gel is filtered and washed, and the solid content is dried at 100 to 200 °C and then calcined at 400 to 900 °C to obtain zeolite powder.

As the silica source used in preparing the raw material gel, one kind or two kinds or more of silicates such as fumed silica, silica sol, silica gel, silicon dioxide, and water glass, silicon alkoxides such as tetraethoxyorthosilicate and tetramethoxysilane, and silicon halides can be used.

As the aluminum source, one kind or two kinds or more of aluminum sulfate, aluminum nitrate, pseudoboehmite, aluminum alkoxide, aluminum hydroxide, alumina sol, sodium aluminate, etc., can be used.

As the gallium source, one kind or two kinds or more of gallium nitrate, gallium sulfate, gallium phosphate, gallium chloride, gallium bromide, gallium hydroxide, etc., can be used.

As the boron source, one kind or two kinds or more of boric acid, sodium borate, boron oxide, etc. can be used.

The molar ratio (Si/Al) of Silicon (Si) to Aluminum (Al) in a raw material gel is not particularly limited, however the lower limit of the molar ratio (Si/Al) of Silicon (Si) to Aluminum (Al) is preferably 200 or more, more preferably 250 or more, further preferably 300 or more. Further, the upper limit of the molar ratio (Si/Al) of silicon (Si) to aluminum (Al) is preferably 4000 or less, more preferably 2000 or less, further preferably 1000 or less. By setting the molar ratio (Si/Al) of silicon (Si) to aluminum (Al) in the raw material gel within the above range, the molar ratio (Si/Al) of silicon (Si) to aluminum (Al) in the synthetic product can be set within a specific range, and a long-life CON-type zeolite can be obtained.

The molar ratio (Si/B) of Silicon (Si) to Boron (B) in a raw material gel is not particularly limited, however the lower limit of the molar ratio (Si/B) of Silicon (Si) to Boron (B) is preferably 5 or more, more preferably 7 or more, further preferably 10 or more. Further, the upper limit of the molar ratio (Si/B) of silicon (Si) to boron (B) is preferably 100 or less, more preferably 75 or less, further preferably 50 or less. By setting the molar ratio (Si/B) of silicon (Si) to boron (B) in the raw material gel within the above range, it becomes easier to direct the synthesis of CON-type zeolite, and the synthesis yield is improved.

The molar ratio (SDA/Si) of the organic structure-directing agent (SDA) to silicon (Si) in the raw material gel is not particularly limited, however the lower limit of the molar ratio (SDA/Si) of the organic structure-directing agent (SDA) to silicon (Si) is preferably 0.05 or more, more preferably 0.10 or more, and further preferably 0.15 or more. The upper limit of the molar ratio (SDA/Si) of the organic structure-directing agent (SDA) to silicon (Si) is preferably 0.50 or less, more preferably 0.40 or less, and further preferably 0.30 or less. By setting the molar ratio (SDA/Si) of the organic structure-directing agent (SDA) to silicon (Si) in the raw material gel within the above range, a highly crystalline CON-type zeolite can be synthesized inexpensively.

The molar ratio (M/Si) of the alkali source (M) to silicon (Si) in the raw material gel is not particularly limited, however the lower limit of the molar ratio (M/Si) of the alkali source (M) to silicon (Si) is preferably 0.05 or more, more preferably 0.07 or more, and further preferably 0.09 or more. The upper limit of the molar ratio (SDA/Si) of the alkali source (M) to silicon (Si) is preferably 0.30 or less, more preferably 0.25 or less, and further preferably 0.20 or less. By setting the molar ratio (M/Si) of alkali source (M) to silicon (Si) in the raw material gel within the above range, a CON-type zeolite can be synthesized without impurities.

The molar ratio (H₂O/Si) of water (H₂O) to silicon (Si) in the raw material gel is not particularly limited, however the lower limit of the molar ratio (H₂O/Si) of water (H₂O) to silicon (Si) is preferably 10 or more, more preferably 13 or more, and further preferably 15 or more. The upper limit of the molar ratio (H₂O/Si) of water (H₂O) to silicon (Si) is preferably 40 or less, more preferably 30 or less, and further preferably 25 or less. By setting the molar ratio (H₂O/Si) of water (H₂O) to silicon (Si) in the raw material gel within the above range, a synthetic gel with an appropriate viscosity can be obtained, and CON-type zeolite having a small particle size and an external surface area can be obtained.

The molar ratio (Si/Al) of silicon (Si) to aluminum (Al) in the CON-type zeolite used as the seed crystals is not particularly limited, and is preferably in the range of 25 to 10,000. The seed crystals are preferably used in an amount of 1 mass% or more, more preferably 2 mass% or more, and further preferably 4 mass% or more, based on the silica source to be added.

For the CON-type zeolite, the amount of metal contained therein can be adjusted by adjusting the amounts of constituent elements (Si, Al, Ga, B, etc.) during synthesis. Furthermore, it is also possible to use those in which the content of the constituent elements has been adjusted by removing a part of the constituent elements by steaming, acid treatment, etc.

In this embodiment, the CON-type zeolite prepared by hydrothermal synthesis may be subjected to an alkali treatment in the presence of a surfactant. By an alkali treatment, the zeolite is meso-structured, and it becomes easier to adjust the average primary particle diameter and the value of (A2/A1) to the desired range.

As the surfactant used in an alkali treatment step, a cationic surfactant, an anionic surfactant, a nonionic surfactant, an amphoteric surfactant, etc., can be used alone or in combination. Examples include hexadecyltrimethylammonium bromide, hexadecyltrimethylammonium chloride, and hexadecyltrimethylammonium hydroxide.

The concentration of the surfactant in the alkali treatment step is not particularly limited, and it may be contained in the treatment liquid at about 0.1 to 10 mass%.

The alkali for the alkali treatment is not particularly limited, and an ammonia solution, a sodium hydroxide solution, etc., can be used, and the degree of alkali is also not particularly limited, and for example, the pH may be about 9 to 12.

The alkali treatment may be carried out by contacting the CON-type zeolite with an alkali, or by stirring in an alkaline solution containing a surfactant. The temperature of the alkali treatment is not particularly limited, and for example, it may be about 100 to 200°C. The time for the alkali treatment is not particularly limited, and it may be immersed in the alkaline solution containing a surfactant for 0.1 to 24 hours.

It is preferable to remove the incorporated surfactant by drying after alkali treatment and calcinating again to be used as a catalyst.

### [Catalyst]

The CON-type zeolite of this embodiment can be used as a catalyst. In particular, the CON-type zeolite of the present embodiment is useful as a catalyst used in a reaction for producing a lower olefin, however, the applications of the zeolite catalyst of the present embodiment are not limited to catalysts for producing a lower olefin, and the catalyst can also be suitably used in the production of p-xylene, the production of ethylbenzene and cumene, the aromatization of light hydrocarbons, hydrocracking, hydrodewaxing, alkane isomerization, and automobile exhaust gas purification.

The CON-type zeolite of the present embodiment may be used as it is in the reaction as a catalyst in the present invention, or may be used as a mixture with other substances inert to the reaction, such as compounds containing alkaline earth metals or silicon, or may be granulated or molded using a binder and used in the reaction as a catalyst containing CON-type zeolite. Examples of the material inactive to the reaction or binder includes alumina or alumina sol, silica, silica gel, silicate, quartz, and mixtures thereof. Among these, silica is preferred from the viewpoint that it is expected to have excellent strength and catalytic performance as an industrial catalyst. Mixing with these substances is also effective in reducing the overall cost of the catalyst, increasing the density of the catalyst, and increasing the strength of the catalyst.

In the method for producing a lower olefin using a catalyst containing the CON-type zeolite of this embodiment, examples of raw materials include methanol and dimethyl ether, however they are not limited to these, and can be appropriately selected depending on the type of target olefin.

The method for producing a lower olefin using the above catalyst will be described below by exemplifying the case where the raw material is methanol and/or dimethyl ether. In this description, a lower olefin means ethylene, propylene and butene. In other words, it is an olefin having a carbon number of 2 to 4.

### [Method for producing lower olefin]

A method for producing a lower olefin of this embodiment of the present invention contains a step of contacting a catalyst containing the CON-type zeolite with a raw material containing methanol or/and dimethyl ether.

The derivation of producing of methanol and dimethyl ether used as raw materials is not particularly limited. Examples include those obtained by hydrogenation reactions of hydrogen/CO mixed gases derived from coal, natural gas, and by-products in the steel industry, those obtained by reforming reactions of plant-derived alcohols, those obtained by fermentation methods, those obtained from organic materials such as recycled plastics and urban waste, and those obtained by methanol synthesis reactions using carbon dioxide as a raw material. At this time, those in a state of mixing compounds other than methanol and dimethyl ether, which are derived from each production method, may be used as they are, or purified.

As the reaction raw material, only methanol or only dimethyl ether may be used, or a combination of these may be used. When methanol and dimethyl ether are used in combination, there is no limitation on the mixing ratio.

The reaction mode in this embodiment is not particularly limited as long as the methanol and/or dimethyl feeding-raw materials are in the gas phase in the reaction zone, and a known gas phase reaction process using a fluidized bed reactor, a moving bed reactor, or a fixed bed reactor can be applied. A fixed bed reactor is advantageous in terms of facility costs including auxiliary facilities, catalyst costs, and operation management.

It can be carried out in batch, semi-continuous, or continuous mode, however it is preferable to be carried out in continuous mode, and the method may be a method using a single reactor or a method using multiple reactors arranged in series or parallel.

When the fixed bed reactor is packed with the above-mentioned catalyst, in order to minimize the temperature distribution in the catalyst layer, granular materials inactive in the reaction, such as quartz sand, alumina, silica, silica-alumina, may be mixed with the catalyst and packed therein. In this case, there is no particular limitation on the amount of granular material such as quartz sand that is inactive in the reaction. From the viewpoint of uniform mixing with the catalyst, it is preferable that the granular material has a particle size approximately equal to that of the catalyst.

In addition, the reaction substrate (reaction raw material) may be divided and fed to the reactor for the purpose of dispersing heat generated by the reaction.

There is no particular limitation on the total concentration (substrate concentration) of methanol and dimethyl ether in all the components fed to the reactor, however the sum of methanol and dimethyl ether is preferably 90 mol% or less in all the components fed. Further preferably, it is 10 mol% or more and 70 mol% or less.

In addition to methanol and/or dimethyl ether, gases inert to the reaction (also referred to as diluents), such as helium, argon, nitrogen, carbon monoxide, carbon dioxide, hydrogen, water, paraffins, hydrocarbons such as methane, aromatic compounds, and mixtures thereof, can be present in the reactor, and among these, the coexistence of water (water vapor) is preferred since it is easily separated.

As such a diluent, impurities contained in the reaction raw materials may be used as they are, or a separately prepared diluent may be used by mixed with the reaction raw materials. The diluent may be mixed with the reaction raw material before introduced into the reactor, or may be fed to the reactor separately from the reaction raw material.

The lower limit of the reaction temperature is usually about 200 °C or higher, preferably 250 °C or higher, more preferably 300 °C or higher, and particularly preferably 400 °C or higher, and the upper limit of the reaction temperature is usually 750 °C or lower, preferably 700 °C or lower, and more preferably 600 °C or lower. By setting the reaction temperature to the above lower limit, the reaction rate is high, the remaining unreacted raw materials can be prevented, and the yield of a lower olefin can be improved. Furthermore, by setting the reaction temperature to the above upper limit or less, stable catalyst activity can be obtained, and a decrease in the yield of a lower olefin can be prevented. Here, the reaction temperature refers to the temperature at the outlet of the catalyst layer.

The upper limit of the reaction pressure is usually 5 MPa (absolute pressure, the same applies below), preferably 2 MPa or less, more preferably 1 MPa or less, more preferably 0.7 MPa or less, and particularly preferably 0.4 MPa or less. The lower limit of the reaction pressure is not particularly limited, and is usually 0.1 kPa or more, preferably 7 kPa or more, and more preferably 50 kPa or more. By setting the reaction pressure to be equal to or lower than the above upper limit, the amount of undesirable by-products such as paraffins and aromatic compounds produced can be suppressed, and the yield of a lower olefin tends to be increased. By setting the reaction pressure to be equal to or higher than the above lower limit, the reaction rate can be increased.

As the reactor outlet gas (reactor effluent), a mixed gas containing a lower olefin which is reaction products, by-products and a diluent can be obtained. The concentration of a lower olefin in the mixed gas is usually 5 to 95 mass%.

Depending on the reaction conditions, methanol and/or dimethyl ether may be contained in the reaction product as unreacted raw materials, and it is preferable to carry out the reaction under reaction conditions such that a conversion rate of methanol and/or dimethyl ether is 100%. This makes separation of the reaction products and the unreacted raw material easy, and preferably makes it unnecessary.

Examples of the by-products include olefins having a carbon number of 5 or more, paraffins, aromatic compounds and water.

A mixed gas containing a lower olefin which is the reaction product, unreacted raw materials, by-products and diluent, as the reactor outlet gas, may be introduced into a known separation and purification facility and may be recovered, purified, recycled or discharged depending on the respective components.

### Example

The present invention will be specifically described below with reference to examples, however the present invention is not limited to these in any way as long as it does not go beyond the gist thereof.

### <Analysis of 8-ketotricyclo[5.2.1.0^{2,6}]decene (manufactured by Aldrich)>

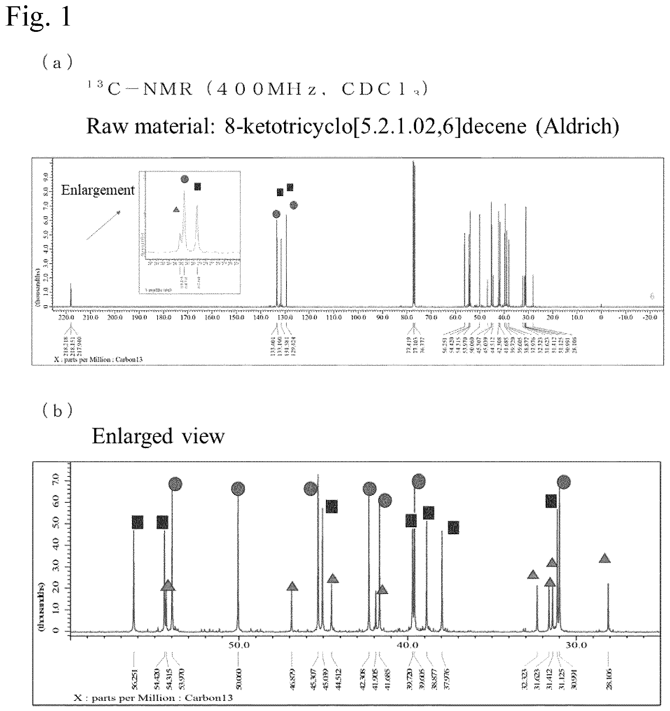

8-Ketotricyclo[5.2.1.0^{2,6}]decene (Aldrich), which is a raw material for the quaternary ammonium salt used in the following Examples 1 and 2, was analyzed by ¹³C-NMR. FIg.1 shows the ¹³C-NMR spectrum. Fig. 1(a) shows the spectrum over the entire measured range, and (b) shows an enlarged view around of 25 ppm to 60 ppm. Table 3 shows the chemical shift values.

From the ¹³C-NMR spectrum, the raw material was a mixture of two isomers, 8-ketotricyclo[5.2.1.0^{2,6}]dec-4-ene (I-K) and 8-ketotricyclo[5.2.1.0^{2,6}]dec-3-ene (II-K), and a small amount of 8-ketotricyclo[5.2.1.0^{2,6}]decane (III-K), shown in the following formulas. In the Examples, the mixture of these three compounds is referred to as 8-ketotricyclo[5.2.1.0^{2,6}]decene.

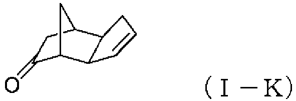

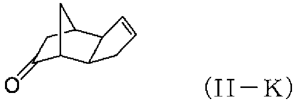

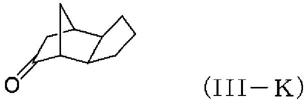

**Table 3**

| ¹³C-NMR chemical shift value (400Mz, CDCl₃) | | | | | |
|---|---|---|---|---|---|
| Reagent operation value ppm (8-ketotricyclo[5.2.1.0^{2,6}]decene) | | | Literature value (ppm) | | |
| Peak ● | Peak ■ | Peak ▲ | 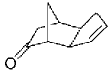 | 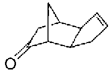 | 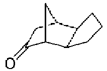 |
| Corresponds to (I-K) | Corresponds to (II-K) | Corresponds to (III-K) | (I-K) | (II-K) | (III-K) |
| 217.5 | 217.9 | 218.2 | 217.5 | 217.3 | 217.2 |
| 133.4 | 133.2 | 54.3 | 133.4 | 133.1 | 54.3 |
| 129.4 | 131.6 | 46.9 | 129.4 | 131.6 | 46.9 |
| 54.0 | 56.3 | 44.5 | 54.0 | 56.2 | 44.5 |
| 50.1 | 54.4 | 41.9 | 50.1 | 54.4 | 41.9 |
| 45.3 | 45.0 | (39.7) | 45.3 | 44.9 | 39.7 |
| 42.3 | 39.7 | 32.3 | 42.3 | 39.7 | 32.3 |
| 41.7 | 38.9 | 31.6 | 41.7 | 38.8 | 31.6 |
| 39.6 | 38.0 | 31.4 | 39.6 | 37.9 | 31.4 |
| 31.0 | 31.4 | 28.1 | 31.0 | 31.1 | 28.1 |

### [Example 1]

### <Production of N,N,N-trimethyl-8-ammonium tricyclo[5.2.1.0^{2,6}]decene hydroxide (TCDEOH-1)>

10.7 g (0.072 mole) of 8-ketotricyclo[5.2.1.0^{2,6}]decene (manufactured by Aldrich: mixture of three types) and 72.2 ml of dimethylamine (2 mole/L methanol solution) were introduced in a three-neck flask, and 6.65 g (0.144 mole) of formic acid was added dropwise over 30 minutes while cooling. After the dropwise addition was completed, it was returned to room temperature, and 79.2 mg (0.144 mmole: 0.2 mole % relative to the substrate ketone) of chloro(pentamethylcyclopentadienyl)(4-dimethylamino-8-quinolinolato)iridium(III) (Kanto Chemical Co., Inc.) was added, followed by stirring at 60 to 65 °C for 5 hours. After confirming that all the raw materials had disappeared by GC, post-processing was performed. For post-processing, after distilling off the solvent, 50 ml of dichloromethane and 25 ml of 10% aqueous sodium hydroxide solution were added, the dichloromethane layer was removed and washed with 50 ml of H₂O, and then the dichloromethane layer was treated with R-Cat-Sil TA (metal scavenger manufactured by Kanto Chemical Co., Inc.). Thereafter, dichloromethane was distilled off to obtain 10.20 g (yield 79.9%) of N,N-dimethylaminotricyclo[5.2.1.0^{2,6}]decene, which was a yellow, slightly viscous liquid. According to ¹³C-NMR, it was a mixture of three types.

10.2 g (0.057 mole) of N,N-dimethylaminotricyclo[5.2.1.0^{2,6}]decene and 100 ml of acetonitrile were charged into a three-neck flask, and then 24.5 g (0.173 mole) of methyl iodide was added dropwise at room temperature. Thereafter, the mixture was heated and stirred at 70 to 75 °C for 2 hours. After confirming that the raw materials had disappeared by GC and ¹H-NMR, the solvent was distilled off, 100 ml of ether and 100 ml of H₂O were added, the H₂O layer was concentrated to its half volume, and ion exchange was carried out with DIAION SA10A (OH type). The aqueous solution after ion exchange was concentrated to obtain 22.1 g of an aqueous solution of N,N,N-trimethyl-8-ammonium tricyclo[5.2.1.0^{2,6}]decene hydroxide (TCDEOH-1). The concentrated aqueous solution was titrated with 0.1 mole hydrochloric acid solution, and it was 45.1% (yield 83.1%) aqueous solution. N,N,N-trimethyl-8-ammonium tricyclo[5.2.1.0^{2,6}]decene hydroxide (TCDEOH-1) was a mixture of three types shown in the following formula. The identification of mixture of three types was determined by two-dimensional NMR analysis.

Since N,N,N-trimethyl-8-ammonium tricyclo[5.2.1.0^{2,6}]decane hydroxide (TCDEOH-III-exo) is a known compound, another synthesis was carried out and used to determine the structures of N,N,N-trimethyl-8-ammonium tricyclo[5.2.1.0^{2,6}]dec-4-ene hydroxide (TCDEOH-I-exo) and N,N,N-trimethyl-8-ammonium tricyclo[5.2.1.0^{2,6}]dec-3-ene hydroxide (TCDEOH-II-exo).

The following NMR chemical shift values indicated the values assigned to each structure based on the results of two-dimensional NMR analysis.

### <N,N,N-trimethyl-8-ammonium tricyclo[5.2.1.0^{2,6}]dec-4-ene hydroxide (TCDEOH-I-exo)>

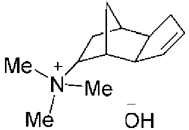

¹H-NMR (600MHz, D₂O) δ (ppm): 1.28, 1.59, 2.18, 2.38, 2.64, 2.70, 3.20 (s, 9H), 3.35 (d, 1H), 3.83 (m, 1H), 5.51 (m, 1H), 5.84 (m, 1H)
¹³C-NMR (600MHz, D₂O) δ (ppm): 31.53, 35.03, 42.52, 45.76, 45.81, 45.98, 50.40, 55.77, 55.79, 55 .82,80.32,133.45,136.66
MS: 192.17 (M⁺)

### <N,N,N-trimethyl-8-ammonium tricyclo[5.2.1.0^{2,6}]dec-3-ene hydroxide (TCDEOH-II-exo)>

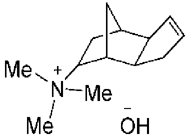

¹H-NMR (600MHz, D₂O) δ (ppm): 1.28, 1.58, 1.97, 2.15, 2.25 (1H), 2.82 (1H), 2.88 (1H),
3.20 (s, 9H), 3.76 (m, 1H), 5.60 (m, 1H), 5.83 (m, 1H)
¹³C-NMR (600MHz, D₂O) δ (ppm): 31.73, 34.67, 38.82, 42.39, 43.67, 48.57, 55.72, 55.77, 55.79, 57.41,79.91, 134.47, 135.93
MS: 192.17 (M⁺)

### <N,N,N-trimethyl-8-ammonium tricyclo[5.2.1.0^{2,6}]decane hydroxide (TCDEOH-III-exo)>

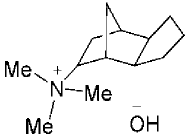

¹H-NMR (600MHz, D₂O) δ (ppm): 1.00 (m, 2H), 1.20 (m, 2H), 1.39 (bd, 1H), 1.62 (d, 1H), 1 .67 (m, 1H), 1.95 (m, 2H), 2.07 (m, 2H), 2.17 (d, 1H), 2.52 (q, 1H), 2.60 (s, 1H), 3.14 (s, 9H), 3.77 (m, 1H)
¹³C-NMR (600MHz, D₂O) δ (ppm): 29.01, 31.58, 34.77, 34.95, 35.39, 42.52, 43.48, 46.68, 49.78, 55.68, 55.70, 55.72, 79.85
MS: 194.19 (M⁺)

### <Structural analysis by NMR>

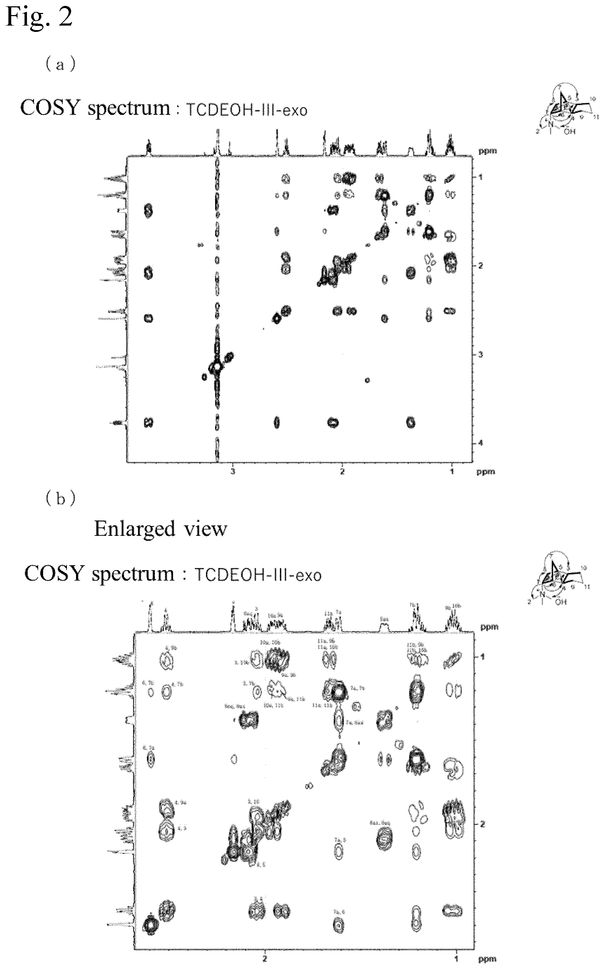

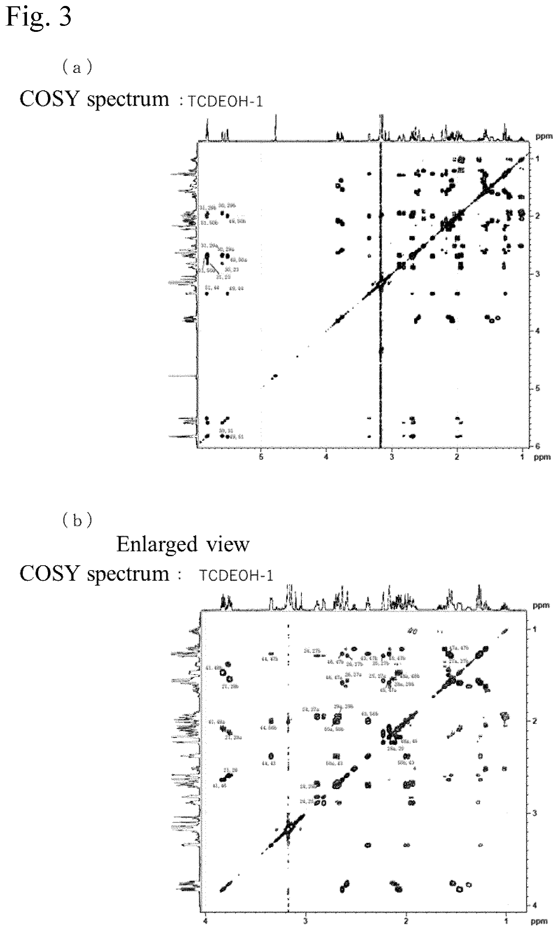

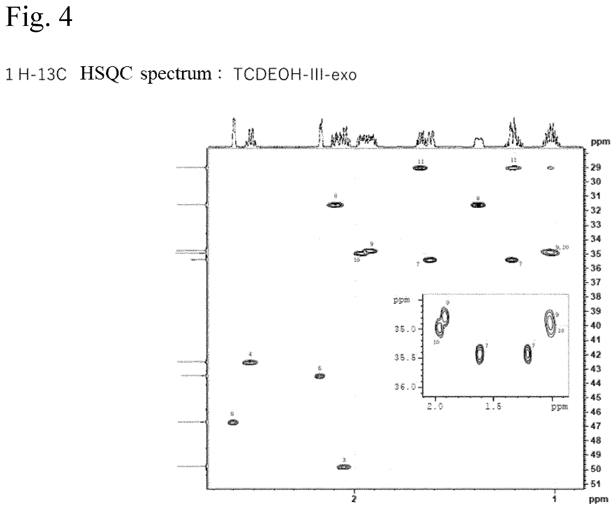

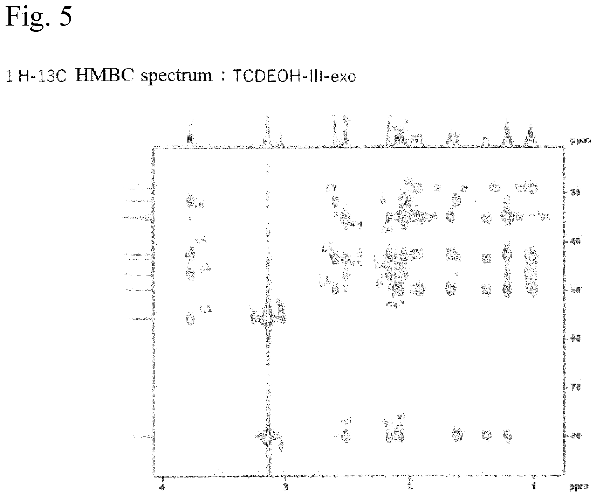

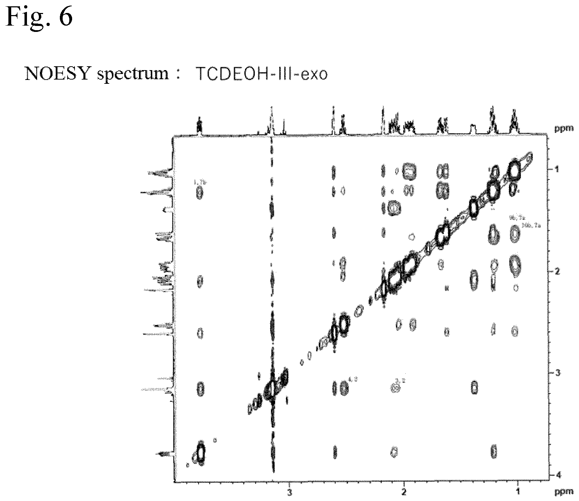

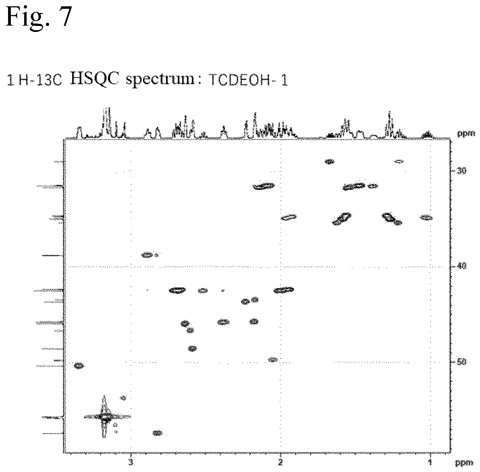

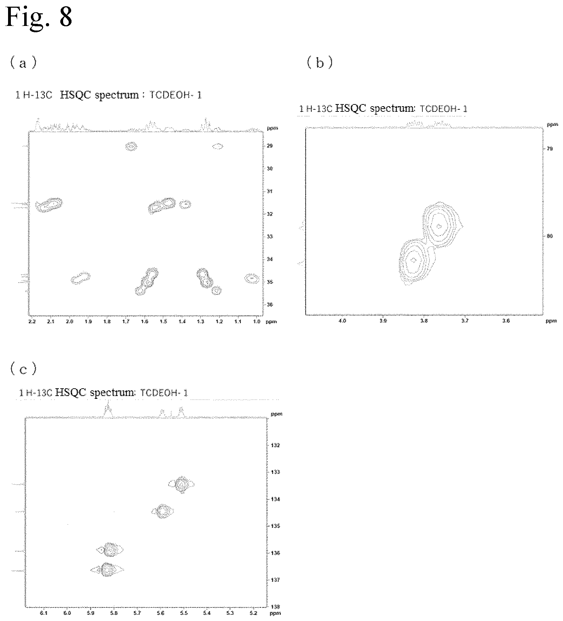

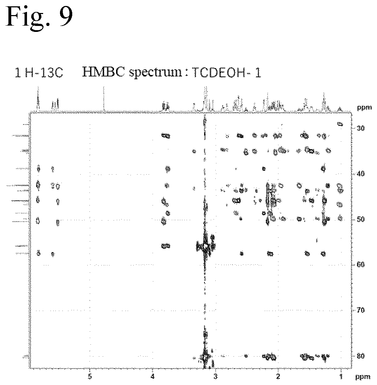

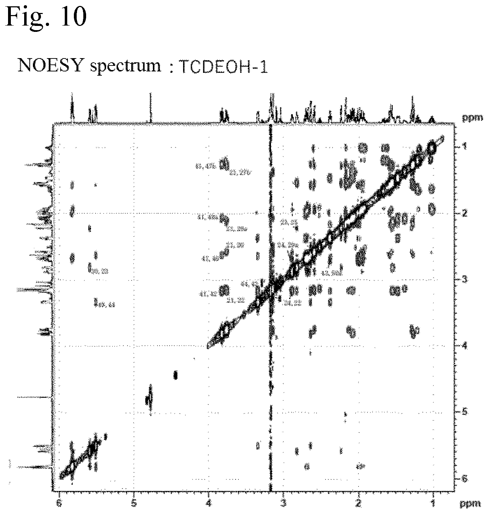

Figs. 2 to 6 show two-dimensional NMR spectra of N,N,N-trimethyl-8-ammonium tricyclo[5.2.1.0^{2,6}]decene hydroxide (TCDEOH-1) and N,N,N-trimethyl-8-ammonium tricyclo[5.2.1.0^{2,6}]decane hydroxide (TCDEOH-III-exo). Fig. 2(a) is a diagram showing the COSY spectrum of N,N,N-trimethyl-8-ammonium tricyclo[5.2.1.0^{2,6}]decane hydroxide (TCDEOH-III-exo), and (b) is an enlarged view. Fig. 3(a) is a diagram showing the COSY spectrum of N,N,N-trimethyl-8-ammonium tricyclo[5.2.1.0^{2,6}]decene hydroxide (TCDEOH-1), and (b) is an enlarged view. Fig. 4 is a diagram showing the HSQC spectrum of N,N,N-trimethyl-8-ammonium tricyclo[5.2.1.0^{2,6}]decane hydroxide (TCDEOH-III-exo). Fig. 5 is a diagram showing the HMBC spectrum of N,N,N-trimethyl-8-ammonium tricyclo[5.2.1.0^{2,6}]decane hydroxide (TCDEOH-III-exo). Fig. 6 is a diagram showing the NOESY spectrum of N,N,N-trimethyl-8-ammonium tricyclo[5.2.1.0^{2,6}]decane hydroxide (TCDEOH-III-exo). Fig. 7 is a diagram showing the HSQC spectrum of N,N,N-trimethyl-8-ammonium tricyclo[5.2.1.0^{2,6}]decene hydroxide (TCDEOH-1). Fig. 8 is a enlarged diagram showing the HSQC spectrum of N,N,N-trimethyl-8-ammonium tricyclo[5.2.1.0^{2,6}]decene hydroxide (TCDEOH-1). Fig. 9 is a diagram showing the HMBC spectrum of N,N,N-trimethyl-8-ammonium tricyclo[5.2.1.0^{2,6}]decene hydroxide (TCDEOH-1). Fig. 10 is a diagram showing the NOESY spectrum of N,N,N-trimethyl-8-ammonium tricyclo[5.2.1.0^{2,6}]decene hydroxide (TCDEOH-1).

### [Example 2]

### <Production of N,N,N-trimethyl-8-ammonium tricyclo[5.2.1.0^{2,6}]decene hydroxide (TCDEOH-2)>

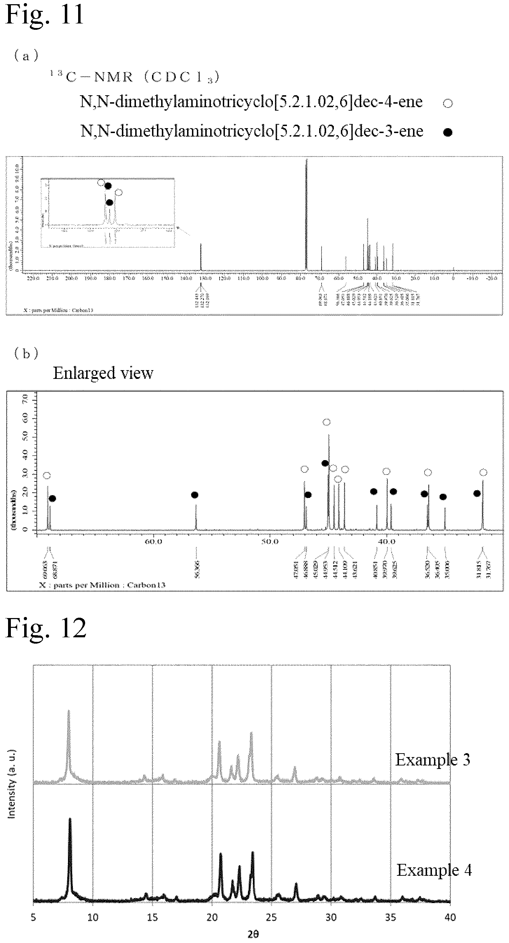

6.55 g (0.044 mole) of 8-ketotricyclo[5.2.1.0^{2,6}]decene (mixture of two types [I-K, II-K) and 44.2 ml of dimethylamine (2 mole/L methanol solution) were introduced in a three-neck flask, and 4.07 g (0.088 mole) of formic acid was added dropwise over 30 minutes while cooling. After the dropwise addition was completed, it was returned to room temperature, and 48.6 mg (0.088 mmol: 0.2 mole % relative to the substrate ketone) of chloro(pentamethylcyclopentadienyl)(4-dimethylamino-8-quinolinolato)iridium(III) (Kanto Chemical Co., Inc.) was added, followed by stirring at 60 to 65 °C for 5 hours. After confirming that all the raw materials had disappeared by GC, post-processing was performed. For post-processing, after distilling off the solvent, 200 ml of dichloromethane and 100 ml of 10% aqueous sodium hydroxide solution were added, the dichloromethane layer was removed and washed with 100 ml of H₂O, and then the dichloromethane layer was treated with R-Cat-Sil TA (metal scavenger manufactured by Kanto Chemical Co., Ltd.). Thereafter, dichloromethane was distilled off to obtain 7.22 g (yield 92.1%) of N,N-dimethylaminotricyclo[5.2.1.0^{2,6}]decene, which was a yellow, slightly viscous liquid. Fig. 11 shows ¹³C-NMR spectrum of N,N-dimethylaminotrycyclo[5.2.1.0^{2,6}]decene. Fig. 11(a) shows the spectrum over the entire measured range, and (b) shows an enlarged view of the vicinity of 30 ppm to 70 ppm. It was a mixture of two types (N,N-dimethylaminotricyclo[5.2.1.0^{2,6}]dec-4-ene and N,N-dimethylaminotricyclo[5.2.1.0^{2,6}]dec-3-ene), by ¹³C-NMR.

3.02 g (0.017 mole) of N,N-dimethylaminotricyclo[5.2.1.0^{2,6}]decene, 4.53 g (0.050 mole) of dimethyl carbonate, and 1.55 g of a mixed solvent of MeOH/H₂O (=3/1) were charged into a microautoclave, sealed, and stirred under slight pressure (up to 2 MPa) at 150 °C for hours.

For the post-processing of the reaction mixture, after the solvent was distilled off, 10 ml of ethyl acetate and 10 ml of H₂O were added, and the H₂O layer was concentrated to half its volume, and ion exchange was carried out with DIAION SA10A (OH type). The aqueous solution after ion exchange was concentrated to obtain 22.1 g of an aqueous solution of N,N,N-trimethyl-8-ammonium tricyclo[5.2.1.0^{2,6}]decene hydroxide (TCDEOH-2).

The concentrated aqueous solution was titrated with 0.1 mole hydrochloric acid solution, and it was 13.6 % (yield 83.1%) aqueous solution. N,N,N-trimethyl-8-ammonium tricyclo[5.2.1.0^{2,6}]decene hydroxide (TCDEOH-2) was a mixture of two types shown in the following formula. The identification of mixture of two types was determined by two-dimensional NMR analysis.

The following NMR chemical shift values indicated the values assigned to each structure based on the results of two-dimensional NMR analysis.

### <N,N,N-trimethyl-8-ammonium tricyclo[5.2.1.0^{2,6}]dec-4-ene hydroxide (TCDEOH-I-exo)>

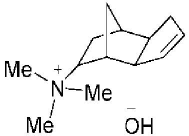

¹H-NMR (400MHz, D₂O) δ (ppm): 1.28, 1.59, 2.18, 2.38, 2.64, 2.70, 3.20 (m, 9H), 3.35 (d, 1H), 3.83 (m, 1H), 5.51 (m, 1H), 5.84 (m, 1H)
¹³C-NMR (400MHz, D₂O) δ (ppm): 31.53, 35.03, 42.52, 45.76, 45.81, 45.98, 50.40, 55.77, 55.79, 55 .82, 80.32, 133.45, 136.66
MS: 192.17 (M⁺)

### <N,N,N-trimethyl-8-ammonium tricyclo[5.2.1.0^{2,6}]dec-3-ene hydroxide (TCDEOH-II-exo)>

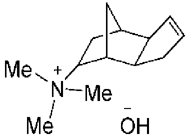

¹H-NMR (600MHz, D₂O) δ (ppm): 1.28, 1.58, 1.97, 2.15, 2.25 (1H), 2.82 (1H), 2.88 (1H), 3.20 (s, 9H), 3.76 (m, 1H), 5.60 (m, 1H), 5.83 (m, 1H)
¹³C-NMR (600MHz, D₂O) δ (ppm): 31.73, 34.67, 38.82, 42.39, 43.67, 48.57, 55.72, 55.77, 55.79, 57.41, 79.91, 134.47, 135.93
MS: 192.17 (M⁺)

### <Production of Zeolite>

### [Example 3]

1.39 g of the aqueous solution of 45.1 mass% N,N,N-trimethyl-8-ammonium tricyclo[5.2.1.0^{2,6}]decene hydroxide produced in Example 1 (hereinafter referred to as TCDEOH-1. Note that the TCDEOH-1 used in Example 3 contains 19% by weight of N,N,N-trimethyl-8-ammonium tricyclo[5.2.1.0^{2,6}]decane hydroxide), 0.097 g of sodium hydroxide, and 4.18 g of demineralized water were mixed, 0.049 g of boric acid (H₃BO₃) and 0.016 g of aluminum sulfate were added to this and stirred, and then 3.93 g of colloidal silica (Cataloid SI-30, manufactured by JGC Catalysts and Chemicals Ltd.) was added as a silica source and thoroughly stirred. Further, 0.120 g of CIT-1 zeolite (Si/Al = 270) was added as seed crystals (Seed) and stirred to prepare a raw material gel.

The obtained raw material gel was charged into an autoclave and heated at 170 °C for 5 days under a rotation speed of 15 rpm to carry out hydrothermal synthesis. The product was filtered, washed with water, and then dried at 100 °C to obtain the zeolite of Example 3 (white powder).

### [Example 4]

4.63 g of the 13.6 mass% N,N,N-trimethyl-8-ammonium tricyclo[5.2.1.0^{2,6}]decene hydroxide (TCDEOH-2) aqueous solution produced in Example 2, 0.100 g of sodium hydroxide, and 0.93 g of demineralized water were mixed, 0.050 g of boric acid (H₃BO₃) and 0.015 g of aluminum sulfate were added and stirred, and then 3.93 g of colloidal silica (Cataloid SI-30, manufactured by JGC Catalysts and Chemicals Ltd.) was added as a silica source and thoroughly stirred. Further, 0.121 g of CIT-1 zeolite (Si/Al = 270) was added as seed crystals (Seed) and stirred to prepare a raw material gel. The obtained raw material gel was heated and post-treated in the same manner as in Example 3 to obtain the zeolite of Example 4 (white powder).

### [Evaluation of zeolite]

### <X-ray diffractometry>

X-ray diffraction measurement of synthesized zeolite was performed using "D2PHASER" manufactured by BRUKER. The XRD pattern obtained by the measurement is shown in Fig. 12. From Fig. 12, it was confirmed that the zeolite in Examples 3 and 4 was SSZ-33.

### <<Method for producing quaternary ammonium salt>>

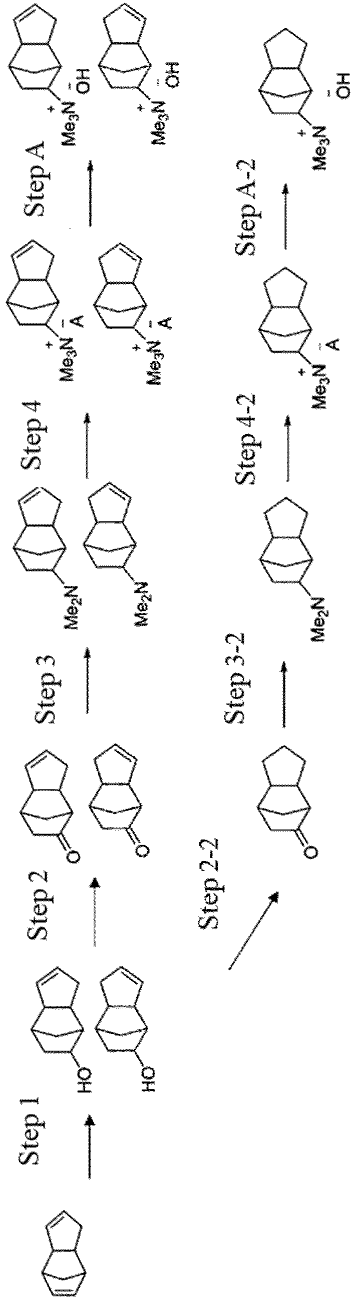

### [Example 5]

In Example 5, N,N,N-trimethyl-8-ammonium tricyclo[5.2.1.0^{2,6}]decene hydroxide (TCDEOH) was produced through steps (1) to (4) and step (A).

### [Method for producing hydroxytricyclo[5.2.1.0^{2,6}]decene: Step (1)]

Into a 300 ml three-neck flask, 53.60 g (0.405 mole) of DCPD and 160.24 g (80.406 mole) of a 25% H₂SO₄ aqueous solution were introduced and stirred at room temperature for 30 minutes, then heated to 100 °C and stirred at 98 to 101.7 °C for 5 hours. After cooling to room temperature, 100 ml of toluene was added and stirred, and the toluene layer was washed with a saturated aqueous solution of Na₂CO₃. The toluene layer after washed was concentrated and then distilled under reduced pressure to obtain 51.64 g (0.344 mole: yield 84.6%) of 8-hydroxytricyclo[5.2.1.0^{2,6}]decene.
b. p. : 103.6-104.3°C/4.5-4.3 Torr

The obtained 8-hydroxytricyclo[5.2.1.0^{2,6}]decene was found to be a mixture of 8-hydroxytricyclo[5.2.1.0^{2,6}]dec-3-ene and 8-hydroxytricyclo[5.2.1.0^{2,6}]dec-4-ene by ¹³C-NMR.

### <8-Hydroxytricyclo[5.2.1.0^{2,6}]dec-3-ene>

¹³C-NMR (400Mz, CDCl₃) δ (ppm): 32.49, 37.90, 39.51, 39.52, 40.44, 49.16, 51.45, 69.61, 130.51, 132.36

### <8-Hydroxytricyclo[5.2.1.0^{2,6}]dec-4-ene>

¹³C-NMR (400Mz, CDCl₃) δ (ppm): 32.17, 35.10, 37.36, 40.19, 40.79, 47.37, 51.30, 71.62, 130.82, 131.63

### [Production of 8-dimethylaminotricyclo[5.2.1.0^{2,6}]decene via 8-ketotricyclo[5.2.1.0^{2,6}]decene: Step (2) to Step (3)]

0.35 g (0.0023 mole) of 8-hydroxytricyclo[5.2.1.0^{2,6}]decene obtained in the step (1), 0.30 g of a copper chromium catalyst (N203SD manufactured by JGC Catalysts and Chemicals Ltd.), and 2 ml of o-xylene were charged into a micro autoclave, and after replacing the atmosphere in the vessel with nitrogen, the mixture was stirred at 200°C for 2 hours in a sealed state.

After the reaction, the reactor was cooled to room temperature, the inside of the vessel was returned to normal pressure, and the vessel was substituted with nitrogen, the reactor was opened, and a portion was taken out as an analytical sample and subjected to GC analysis. As a result, it was found that 8-ketotricyclo[5.2.1.0^{2,6}]decene was produced in a yield of 91.3%. Without removing the reaction solution from the micro autoclave, 4.0 ml of dimethylamine (2 mole/L methanol solution) was charged into the reactor, and then after replacing with nitrogen, H₂ was pressurized to 2.1 MPa and the reaction was carried out at 120 °C for 1 hour.

After the reaction, the catalyst was filtered off from the reaction solution, and the solvent was distilled off to obtain 0.35 g (0.0020 mole: yield 87.0%) of a pale yellow liquid, 8-dimethylaminotricyclo[5.2.1.0^{2,6}]decene.

The obtained 8-dimethylaminotricyclo[5.2.1.0^{2,6}]decene was found to be a mixture of 8-dimethylaminotricyclo[5.2.1.0^{2,6}]dec-3-ene and 8-dimethylmethyltricyclo[5.2.1.0^{2,6}]dec-4-ene by ¹³C-NMR.

### <8-Dimethylaminotricyclo[5.2.1.0^{2,6}]dec-3-ene>

¹³C-NMR (400Mz, CDCl₃) δ (ppm): 31.82, 35.01, 36.52, 39.63, 40.85, 45.03, 46.89, 56.37, 68.87, 132.27, 132.42

### <8- Dimethylaminotricyclo[5.2.1.0^{2,6}]dec-4-ene>

¹³C-NMR (400Mz, CDCl₃) δ (ppm): 31.77, 36.41, 39.97, 43.62, 44.11, 44.51, 44.95, 47.05, 69.06, 132.07, 132.44

### [Production of N,N,N-trimethyl-8-ammonium tricyclo[5.2.1.0^{2,6}]decene iodide: step (4)]

1.70 g (0.0096 mole) of N,N-dimethylaminotricyclo[5.2.1.0^{2,6}]decene produced by the method in step (3) above and 15 ml of acetone were charged into a three-neck flask, and then 2.84 g (0.200 mole) of methyl iodide was added dropwise at room temperature while stirring. Thereafter, the solid precipitated during heating and stirring at 45 to 55°C for 2 hours. After confirming that the raw materials had disappeared by GC and ¹H-NMR, the solid was filtered off, washed with 2 ml of acetone, and vacuum dried to obtain 2.81 g (0.0087 mole, yield: 91.0%) of N,N,N-trimethyl-8-ammonium tricyclo[5.2.1.0^{2,6}]decene iodide as a white to pale yellow solid.

The obtained N,N,N-trimethyl-8-ammonium tricyclo[5.2.1.0^{2,6}]decene iodide was found to be a mixture of N,N,N-trimethyl-8-ammonium tricyclo[5.2.1.0^{2,6}]dec-3-ene iodide and N,N,N-trimethyl-8-ammonium tricyclo[5.2.1.0^{2,6}]dec-4-ene iodide by ¹³C-NMR.

### <N,N,N-trimethyl-8-ammonium tricyclo[5.2.1.0^{2,6}]dec-3-ene iodide: Analytical data>

¹³C-NMR (400Mz, CDCl₃) δ (ppm): 31.80, 34.74, 38.87, 42.46, 43.72, 48.62, 55.78, 55.84, 55.88, 57.47, 79.97, 134.50, 135.97

### <N,N,N-trimethyl-8-ammonium tricyclo[5.2.1.0^{2,6}]dec-4-ene iodide>

¹³C-NMR (400Mz, CDCl₃) δ (ppm): 31.59, 35.09, 42.57, 45.81, 45.86, 46.03, 50.44, 55.78, 55.84, 55.88, 80.37, 133.47, 136.74

### [Conversion to N,N,N-trimethyl-8-ammonium tricyclo[5.2.1.0^{2,6}]decene hydroxide: step (A)]

A mixture of 15.01 g of N,N,N-trimethyl-8-ammonium tricyclo[5.2.1.0^{2,6}]dec-3-ene iodide and N,N,N-trimethyl-8-ammonium tricyclo[5.2.1.0^{2,6}]dec-4-ene iodide synthesized by the above method was subjected to ion exchange using DIAION SA10A (OH type). The aqueous solution after ion exchange was concentrated to obtain 28.02 g of an aqueous solution of N,N,N-trimethyl-8-ammonium tricyclo[5.2.1.0^{2,6}]decene hydroxide (TCDEOH). The concentrated aqueous solution was titrated with 0.1 mole hydrochloric acid solution, and it was 30.1 % (yield 85.7%) aqueous solution. N,N,N-trimethyl-8-ammonium tricyclo[5.2.1.0^{2,6}]decene hydroxide (TCDEOH) was a mixture of two types shown in the following formula by ¹³C-NMR.

### N,N,N-trimethyl-8-ammonium tricyclo[5.2.1.0^{2,6}]dec-3-ene hydroxide (TCDEOH-II)

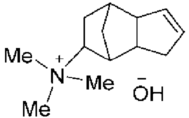

¹H-NMR (400MHz, D₂O) δ (ppm): 1.28, 1.58, 1.97, 2.15, 2.25 (1H), 2.82 (1H), 2.88 (1H) 3.20 (s, 9H), 3.76 (m, 1H), 5.60 (m, 1H), 5.83 (m, 1H)
¹³C-NMR (400MHz, D₂O) δ (ppm): 31.65, 34.63, 38.76, 42.35, 43.61, 48.50, 55.67 (br), 57.35, 79.78, 134.40, 136.57

### N,N,N-trimethyl-8-ammonium tricyclo[5.2.1.0^{2,6}]dec-4-ene hydroxide (TCDEOH-I)

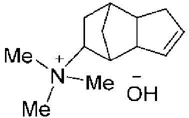

¹H-NMR (400MHz, D₂O) δ (ppm): 1.28, 1.59, 2.18, 2.38, 2.64, 2.70, 3.20, 3.35 (d, 1H), 3.83 (m, 1H), 5.51 (m, 1H), 5.84 (m, 1H)
¹³C-NMR (600MHz, D₂O) δ (ppm): 31.45, 34.99, 42.49, 45.70, 45.74, 45.90, 50.34, 55.68 (br), 80.20, 133.41, 136.57

### [Example 6]

In Example 6, N,N,N-trimethyl-8-ammonium tricyclo[5.2.1.0^{2,6}]decane hydroxide (TCDEOH-III) was produced through step (1), steps (2-2) to (4-2), and step (A). The 8-hydroxytricyclo[5.2.1.0^{2,6}]decene produced in step (1) was the same as that described in Example 5, and therefore the description here is omitted.

### [Production of 8-ketotricyclo[5.2.1.0^{2,6}]decane: step (2-2)]

1.10 g (0.0073 mole) of 8-hydroxytricyclo[5.2.1.0^{2,6}]decene obtained in the step (1) of Example 5, Raney Nickel (Nikko Rica Corporation R200), and 7 ml of o-xylene were charged into a micro autoclave, and after replacing the atmosphere in the vessel with nitrogen, the mixture was stirred at 170°C for 2 hours in a sealed state.

After the reaction, the reactor was cooled to room temperature, returned to normal pressure, and replaced with nitrogen. Thereafter, the reaction solution was taken out, the catalyst was filtered off, and the solvent was distilled off to obtain 1.07 g (0.0072 mole: yield 98.0%) of colorless and transparent 8-ketotricyclo[5.2.1.0^{2,6}]decane.

### [Production of dimethylaminotricyclo[5.2.1.0^{2,6}]decane: step (3-2)]

0.52 g (0.0035 mole) of 8-ketotricyclo[5.2.1.0^{2,6}]decane obtained by the above method and 10.0 ml of dimethylamine (2 mole/L methanol solution) were charged into a micro autoclave, and after replacing the atmosphere in the vessel with nitrogen, H₂ was introduced until the pressure reached 2.1 MPa, and the mixture was stirred at 150°C for 2 hours. After the reaction, the reactor was cooled to room temperature, returned to normal pressure, and replaced with nitrogen. Thereafter, the reaction solution was taken out, the catalyst was filtered off, and the solvent was distilled off to obtain 0.57 g (0.0032 mole: yield 90.6%) of N,N-dimethylaminotricyclo[5.2.1.0^{2,6}]decane as a yellow, slightly viscous liquid.

### [Production of N,N,N-trimethyl-8-ammonium tricyclo[5.2.1.0^{2,6}]decane iodide: step (4-2)]

After charging several portions of N,N-dimethylaminotricyclo[5.2.1.0^{2,6}]decane obtained by the method of step (3-2), 4.37 g (0.0225 mole) and 20 ml of acetone into a three-neck flask, a solution in which 3.79 g (0.0267 mole) methyl iodide dissolved in 10 ml of acetone was added dropwise at room temperature while stirring. Thereafter, the mixture was stirred under reflux, and a solid gradually precipitated. After 2 hours, it was confirmed that the raw materials had disappeared by GC and ¹H-NMR of the solution portion, and then the reaction solution was returned to room temperature, filtered, and vacuum dried at 80°C to obtain a whitish yellow to white solid, N,N,N-trimethyl-8-ammonium tricyclo[5.2.1.0^{2,6}]decane iodide 6.50 g (0.0232 mole: yield 90.0%).

### [Conversion to N,N,N-trimethyl-8-ammonium tricyclo[5.2.1.0^{2,6}]decane hydroxide: step (A-2)]

6.50 g of N,N,N-trimethyl-8-ammonium tricyclo[5.2.1.0^{2,6}]decane iodide synthesized by the above method was dissolved in 20 ml of H₂O and ion-exchanged using DIAION SA10A (OH type), and after the resulting solution was concentrated, 14.16 g of an aqueous solution of N,N,N-trimethyl-8-ammonium tricyclo[5.2.1.0^{2,6}]decane hydroxide, (TCDEOH-III), was obtained. The concentrated aqueous solution was titrated with 0.1 mole hydrochloric acid solution, and it was 31.8 % (yield 91.8%) aqueous solution. The ¹H-NMR and ¹³C-NMR of the obtained N,N,N-trimethyl-8-ammonium tricyclo[5.2.1.0^{2,6}]decane hydroxide (TCDEOH-III) were as follows.

### N,N,N-trimethyl-8-ammonium tricyclo[5.2.1.0^{2,6}]decane hydroxide (TCDEOH-III)

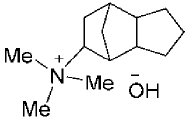

¹H-NMR (400MHz, D₂O) δ (ppm): 1.00 (m, 2H), 1.20 (m, 2H), 1.39 (bd, 1H), 1.62 (d, 1H), 1.67 (m, 1H), 1.95 (m, 2H), 2.07 (m, 2H), 2.17 (d, 1H), 2.52 (q, 1H), 2.60 (s, 1H), 3.14 (s, 9H), 3.77 (m, 1H)
¹³C-NMR (400MHz, D₂O) δ (ppm): 29.01, 31.58, 34.77, 34.95, 35.39, 42.52, 43.48, 46.68, 49.78, 55.68, 55.70, 55.72, 79.85

### [Example 7]

In Example 7, N,N,N-trimethyl-8-ammonium tricyclo[5.2.1.0^{2,6}]decane hydroxide (TCDEOH-III) was produced through step (1), steps (2-2) to (4-2), and step (A-2). The 8-hydroxytricyclo[5.2.1.0^{2,6}]decene produced in step (1) was the same as that described in Example 5, and therefore the description here is omitted. The steps (2-2) and (3-2) were carried out using the same catalyst and in the same reactor.

### [Production of 8-dimethylaminotricyclo[5.2.1.0^{2,6}]decene via 8-ketotricyclo[5.2.1.0^{2,6}]decane: Steps (2-2) to (3-2)]

0.55 g (0.0037 mole) of 8-hydroxytricyclo[5.2.1.0^{2,6}]decene obtained in the step (1) of Example 5, 0.55g of Raney Nickel (R200 prodcued by Nikko Rica Corporation), and 4 ml of toluene were charged into a micro autoclave, and after replacing the atmosphere in the vessel with nitrogen, the mixture was stirred at 180°C for 2 hours in a sealed state.

After the reaction, the reactor was cooled to room temperature, the inside of the vessel was returned to normal pressure, and the vessel was substituted with nitrogen, the reactor was opened, and a portion was taken out as an analytical sample and subjected to GC analysis. As a result, it was found that 8-ketotricyclo[5.2.1.0^{2,6}]decane was produced in a yield of 84.5%. Without removing the reaction solution from the micro autoclave, 6.0 ml of dimethylamine (2 mole/L methanol solution) was charged into the reactor, and then after replacing with nitrogen, H₂ was pressurized to 2.0 MPa and the reaction was carried out at 120 °C for 2 hours.

After the reaction, the reactor was cooled, returned to normal pressure, and replaced with N₂, the reaction solution was then taken out, the catalyst was filtered off, and the solvent was distilled off to obtain 0.48 g (0.0027 mole: yield 72.9%) of a pale yellow liquid, dimethylaminotricyclo[5.2.1.0^{2,6}]decane.

### [Production of N,N,N-trimethyl-8-ammonium tricyclo[5.2.1.0^{2,6}]decane iodide: step (4-2)]

0.48 g (0.0027 mole) of N,N-dimethylaminotricyclo[5.2.1.0^{2,6}]decane obtained by the step 2-2 and the step 3-2 was subjected to the same manner as in step (4-2) described in Example 6 to obtain a whitish yellow to white solid, N,N,N-trimethyl-8-ammoniumtricyclo[5.2.1.0^{2,6}]decane iodide (0.80 g, 0.0025 mole: yield 92.6%).

### [Conversion to N,N,N-trimethyl-8-ammonium tricyclo[5.2.1.0^{2,6}]decane hydroxide: step (A-2)]

0.80 g (0.0025 mole) of N,N,N-trimethyl-8-ammonium tricyclo[5.2.1.0^{2,6}]decane iodide synthesized by the above method was subjected to the same procedure as in step (A-2) described in Example 2 to obtain 1.50 g of an aqueous solution of N,N,N-trimethyl-8-ammonium tricyclo[5.2.1.0^{2,6}]decane hydroxide (TCDOH). This aqueous solution was titrated with 0.1 mole hydrochloric acid solution, and it was 32.6 % (yield 92.7%) aqueous solution.

### <<CON-type zeolite>>

### [Example 8]

1.71 g of the aqueous solution of 36.6 mass% N,N,N-trimethyl-8-ammonium tricyclo[5.2.1.0^{2,6}]decane hydroxide (structure is represented by the following general formula (VI), hereinafter referred to as "TCDOH"), 0.098 g of sodium hydroxide, and 3.81 g of demineralized water were mixed, 0.050 g of boric acid (H₃BO₃) and 0.015 g of aluminum sulfate were added to this and stirred, and then 3.93 g of colloidal silica (Cataloid SI-30, manufactured by JGC Catalysts and Chemicals Ltd.) was added as a silica source and thoroughly stirred. Further, 0.120 g of CIT-1 zeolite (Si/Al = 270) was added as seed crystals (Seed) and stirred to prepare a raw material gel.

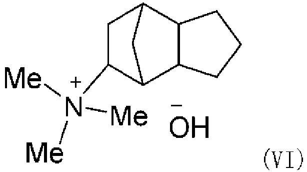

The obtained raw material gel was charged into an autoclave and heated at 170 °C for 5 days under a rotation speed of 15 rpm to carry out hydrothermal synthesis. The product was filtered, washed with water, and then dried at 100 °C to obtain white powder. From the X-ray diffraction (XRD) pattern of the product, it was confirmed that the obtained product was SSZ-33 zeolite. After drying, the mixture was calcined at 600 °C for 6 hours in an air atmosphere to obtain a sodium type zeolite powder.

The obtained powder was subjected to ion exchange in a 1 N aqueous solution of ammonium nitrate at 80 °C for 1 hour, and then filtered and dried. The dried powder was again subjected to ion exchange in a 1N aqueous solution of ammonium nitrate at 80 °C for 1 hour, and then filtered and dried to obtain an ammonium type zeolite powder. Thereafter, the mixture was calcined at 500 °C for 6 hours in an air atmosphere to obtain the proton type zeolite powder of Example 1.

### [Example 9]

1.39 g of the aqueous solution of 45.1 mass% N,N,N-trimethyl-8-ammonium tricyclo[5.2.1.0^{2,6}]decane hydroxide (it is a mixture of structure represented by the following general formulas (IV) and (V), hereinafter referred to as "TCDOH"). Note that the TCDEOH used in Example 9 contained 19% by weight of TCDOH), 0.097 g of sodium hydroxide, and 4.18 g of demineralized water were mixed, 0.049 g of boric acid (H₃BO₃) and 0.016 g of aluminum sulfate were added to this and stirred, and then 3.93 g of colloidal silica (Cataloid SI-30, manufactured by JGC Catalysts and Chemicals Ltd.) was added as a silica source and thoroughly stirred. Further, 0.120 g of CIT-1 zeolite (Si/Al = 270) was added as seed crystals (Seed) and stirred to prepare a raw material gel. The obtained raw material gel was heated and post-treated in the same manner as in Example 1 to obtain the proton type zeolite powder of Example 9.

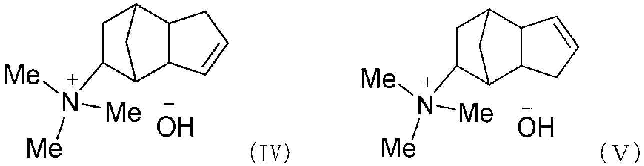

### [Example 10]

10.26 g of a 20.6 mass% TCDOH aqueous solution, 0.308 g of sodium hydroxide, and 6.93 g of demineralized water were mixed, to which 0.2473 g of boric acid (H₃BO₃) and 0.092 g of aluminum sulfate were added and stirred, and then 19.64 g of colloidal silica (Cataloid SI-30, manufactured by JGC Catalysts and Chemicals Ltd.) was added as a silica source and thoroughly stirred. Further, 0.241 g of CIT-1 zeolite (Si/Al = 270) was added as seed crystals (Seed) and stirred to prepare a raw material gel. From the obtained raw material gel, 15 g was taken and heated and post-treated in the same manner as in Example 1, except that it was heated at 185 °C for 25.5 hours, to obtain a proton-type borosilicate zeolite powder.

### [Comparative Example 1]

1.74 g of a 36.6 mass% TCDOH aqueous solution, 0.097 g of sodium hydroxide, and 3.82 g of demineralized water were mixed, 0.050 g of boric acid (H₃BO₃) was added to this and stirred, and then 3.94 g of colloidal silica (Cataloid SI-30, manufactured by JGC Catalysts and Chemicals Ltd.) was added as a silica source and thoroughly stirred. Further, 0.120 g of CIT-1 zeolite (Si/Al = 270) was added as seed crystals (Seed) and stirred to prepare a raw material gel. The obtained raw material gel was heated and post-treated in the same manner as in Example 1 to obtain the proton-type borosilicate zeolite powder.

As a post-processing, 0.403 g of the obtained powder was added to an aqueous solution in which 0.200 g of aluminum nitrate nonahydrate was dissolved in 19.99 g of water, and the mixture was stirred under reflux at 100°C for 8 hours. Thereafter, the zeolite was then filtered, washed with water, and dried at 100 °C overnight. Thereafter, the mixture was calcined at 540 °C for 6 hours in an air atmosphere to obtain the proton type zeolite powder of Comparative example 1.

### [Comparative example 2]

As a post-processing, 0.400 g of the proton-type borosilicate zeolite powder obtained in Comparative Example 1 was added to an aqueous solution in which 0.030 g of aluminum nitrate nonahydrate was dissolved in 19.98 g of water, and the mixture was stirred under reflux at 100 °C for 7 hours. Thereafter, the zeolite was then filtered, washed with water, and dried at 100 °C overnight. Thereafter, the mixture was calcined at 540 °C for 6 hours in an air atmosphere to obtain the proton type zeolite powder of Comparative example 2.

### [Comparative example 3]

30.9 mass% N,N,N-trimethyl-(-)-cis-miltanyl ammonium hydroxide (hereinafter referred to as "TMMAOH"). 2.06 g of the aqueous solution, 0.049 g of sodium hydroxide, and 3.50 g of demineralized water were mixed, to which 0.049 g of boric acid (H₃BO₃) and 0.015 g of aluminum sulfate were added and stirred, and then 3.92 g of colloidal silica (Cataloid SI-30, manufactured by JGC Catalysts and Chemicals Ltd.) was added as a silica source and thoroughly stirred. Further, 0.121 g of CIT-1 zeolite (Si/Al = 270) was added as seed crystals (Seed) and stirred to prepare a raw material gel. The obtained raw material gel was heated and post-treated in the same manner as in Example 1 to obtain the proton type zeolite powder of Comparative example 3.

The synthesis conditions for Examples 8 to 10 and Comparative Examples 1 to 3 are summarized in Table 4. The synthesis was performed by adjusting the molar ratio of the organic structure directing agent (SDA) to Si uniform in Examples 8 and 9 and Comparative Examples 1 to 3, and for Example 10, the synthesis was performed by reducing amount of the organic structure-directing agent added.

### [Evaluation of zeolite]

The zeolites according to Examples 8 to 10 and Comparative Examples 1 to 3 were evaluated as follows.

### <X-ray diffractometry>

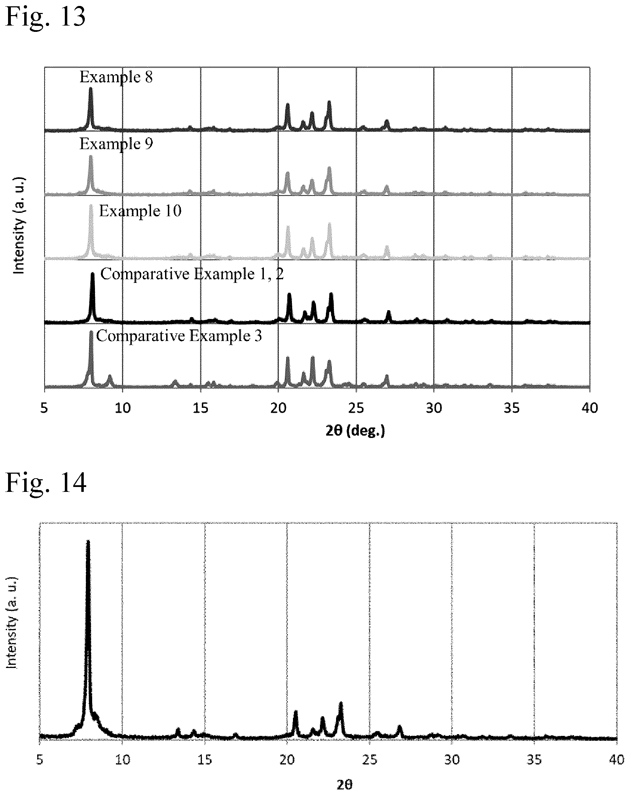

X-ray diffraction (XRD) measurement of synthesized zeolite (as-made type, H type) was performed using "D2PHASER" manufactured by BRUKER. The XRD pattern obtained by the measurement is shown in Fig. 13 and Fig. 14. Fig. 13 is a diagram showing the results of powder X-ray diffraction of the zeolites (as-made type) according to Examples 8 to 10 and Comparative Examples 1 to 3. Fig. 14 is a diagram showing the powder X-ray diffraction results of the zeolite (H type) of Example 9. From Fig. 13 and Fig.14, it was confirmed that the zeolites of Examples 8 to 10 and Comparative Examples 1 and 2 were SSZ-33 zeolites containing 25 mass% or more and 35 mass% or less of Polymorph A, and the zeolite of Comparative Example 3 was CIT-1 zeolite substantially containing Polymorph B.

### <Elemental Analysis>

Elemental analysis was performed by inductively coupled plasma atomic emission spectrometry (ICP-AES). The zeolites of Examples 8 to 10 and Comparative Examples 1 to 3 were measured using "iCAP7600Duo" manufactured by Thermo Fisher Scientific. The Si/Al molar ratios of the synthesized zeolites are shown in Table 4.

### <Scanning Electron Microscope>

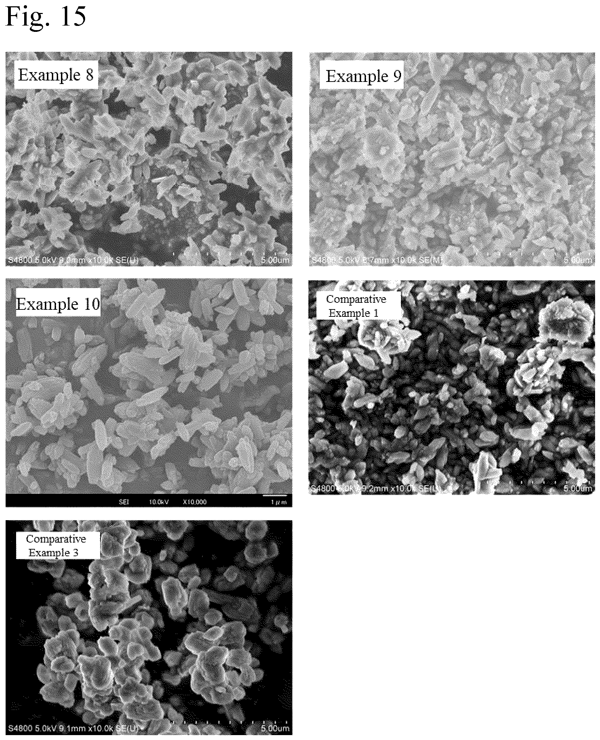

The scanning electron microscope (SEM) measurements for Examples 8 to 9 and Comparative Examples 1 and 3 were carried out using an "S-4800" manufactured by Hitachi High-Technologies Corporation. The SEM measurement in Example 10 was carried out using a JEOL Ltd. "JSM-6701F". Fig. 15 shows SEM images of the zeolite catalysts according to Examples 8 to 10 and Comparative Examples 1 and 3. Fifty primary particles were randomly selected from the obtained SEM image, and the major axis of each particle was measured to determine the particle size. The arithmetic average of the determined particle sizes was taken as the average primary particle diameter. The results are shown in Table 4. Since Comparative Example 2 only differs from Comparative Example 1 in the post-processing process, the average primary particle diameter of Comparative Example 2 was determined to be the same as that of Comparative Example 1.

### <Nitrogen adsorption/desorption measurement>

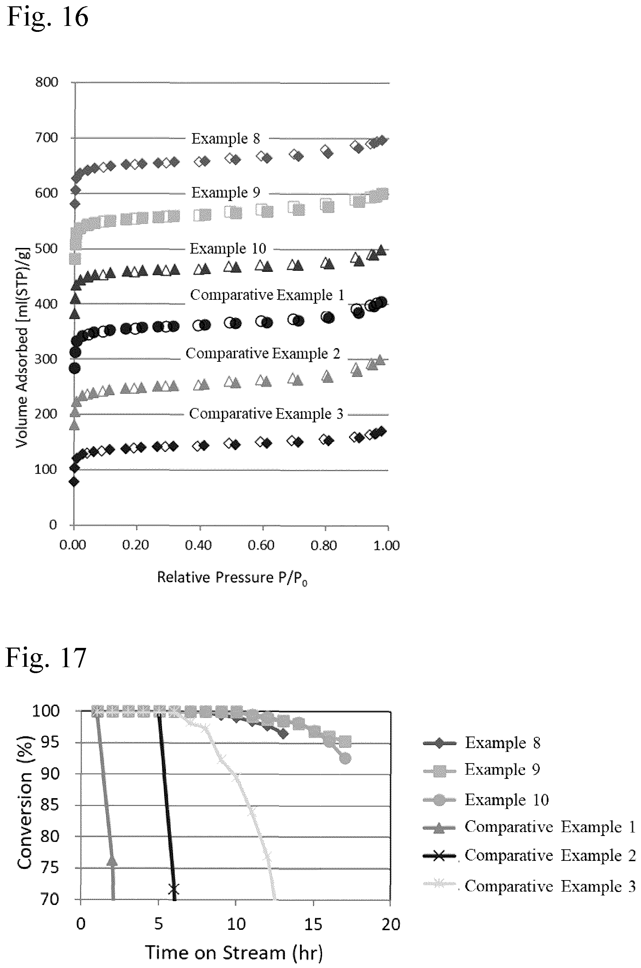

Nitrogen adsorption/desorption measurements of the synthesized proton type zeolite powder were carried out using a Belsorp-mini II manufactured by MicrotracBEL Corp. For the measurement, the zeolite was heated and dried at 400 °C for 2 hours under vacuum, and then nitrogen adsorption/desorption measurements were carried out at liquid nitrogen temperature. Fig. 16 is a graph showing adsorption/desorption isotherms of the zeolites according to Examples 8 to 10 and Comparative Examples 1 to 3. The data analysis was performed using analysis software BELMaster manufactured by MicrotracBEL Corp. The BET specific surface area (A2) was calculated by BET plotting of the measurement data with a relative pressures (P/P₀) of 0.002 to 0.06. The external surface area (A1) and the micropore volume (A3) was calculated by t-plotting of the data with a relative pressures (P/P₀) of 0.20 to 0.42. Note that Harkins-Jura was used as a standard isotherm.

### <Producing lower olefin>

Using the zeolites obtained in Examples 8 to 10 and Comparative Examples 1 to 3, a lower olefin was produced. For the reaction, a fixed bed flow reactor was used, and a quartz reaction tube having an inner diameter of 6 mm was filled with 33 mg of premixed proton type zeolite powder and 470 mg of quartz sand. A mixed gas of 50 mol% methanol and 50 mol% nitrogen was fed to the reactor so that the weight space velocity of methanol was 15 hr⁻¹, and the reaction was carried out at 500 °C and 0.1 MPa (absolute pressure). The products were analyzed by gas chromatography every hour from the start of the reaction. Fig. 17 is a graph showing the change in methanol conversion rate. Table 4 shows the methanol conversion (%) after 2 hours of reaction, the ethylene selectivity (C-mol%), the propylene selectivity (C-mol%), the butene selectivity (C-mol%) and the catalytic life (hr). The catalytic life was defined as the time during which the methanol conversion rate was maintained at 98% or more.

**Table 4**

| | | Example 8 | Example 9 | Example 10 |
|---|---|---|---|---|
| Charged composition (Molar ratio) | SDA type | TCDOH | TCDEOH | TCDOH |
| | SDA/Si | 0.15 | 0.15 | 0.10 |
| | NaOH/Si | 0.15 | 0.15 | 0.10 |
| | H₂O/Si | 21 | 21 | 16 |
| | H₃BO₃/Si | 0.04 | 0.04 | 0.04 |
| | Al₂(SO₄)₃/Si | 0.00125 | 0.00125 | 0.00143 |
| | Seed/Si | 0.10 | 0.10 | 0.04 |
| Post-processing composition (weight ratio) | H₂O/SiO₂ | - | - | - |
| | Al(NO₃)₃·9H₂O/SiO₂ | - | - | - |
| XRD analysis results | Production Phase | SSZ-33 | SSZ-33 | SSZ-33 |
| Elemental analysis result | Si/Al | 332 | 344 | 309 |
| | Si/B | unanalyzed | unanalyzed | 47 |
| SEM analysis results | Particle size (nm) | 730 | 570 | 620 |
| Nitrogen adsorption/desorption determination result | External surface area A1 (m²/g) | 63 | 75 | 51 |
| | BET specific surface area A2 (m²/g) | 598 | 603 | 629 |
| | A2/A1 | 9.5 | 8.0 | 12.3 |
| | Micropore volume A3(m³/g) | 0.210 | 0.208 | 0.227 |
| Catalyst performance | Methanol conversion rate (%) | 100.0 | 100.0 | 100.0 |
| | Ethylene selectivity (C-mol%) | 3.0 | 2.6 | 3.1 |
| | Propylene selectivity (C-mol%) | 48.3 | 49.1 | 49.9 |
| | Butene selectivity (C-mol%) | 21.8 | 22.3 | 23.6 |
| | Catalytic life (hr) | 11 | 14 | 14 |

**Table 4 (continued)**

| | | Comparative Example 1 | Comparative Example 2 | Comparative Example 3 |
|---|---|---|---|---|
| Charged composition (Molar ratio) | SDA type | TCDOH | TCDOH | TMMAOH |
| | SDA/Si | 0.15 | 0.15 | 0.15 |
| | NaOH/Si | 0.15 | 0.15 | 0.09 |
| | H₂O/Si | 21 | 21 | 21 |
| | H₃BO₃/Si | 0.04 | 0.04 | 0.04 |
| | Al₂(SO₄)₃/Si | 0 | 0 | 0.00125 |
| | Seed/Si | 0.10 | 0.10 | 0.10 |
| Post-processing composition (weight ratio) | H₂O/SiO₂ | 167 | 167 | - |
| | Al(NO₃)₃·9H₂O/SiO₂ | 0.080 | 0.012 | - |
| XRD analysis results | Production Phase | SSZ-33 | SSZ-33 | CIT-1 |
| Elemental analysis result | Si/Al | 60 | 82 | 345 |
| | Si/B | 487 | 630 | unanalyzed |
| SEM analysis results | Particle size (nm) | 640 | 640 | 550 |
| Nitrogen adsorption/desorption determination result | External surface area A1 (m²/g) | 61 | 66 | 54 |
| | BET specific surface area A2 (m²/g) | 613 | 577 | 547 |
| | A2/A1 | 10.0 | 8.7 | 10.1 |
| | Micropore volume A3(m³/g) | 0.218 | 0.202 | 0.194 |
| Catalyst performance | Methanol conversion rate (%) | 76.3 | 100.0 | 100.0 |
| | Ethylene selectivity (C-mol%) | 4.2 | 7.4 | 2.7 |
| | Propylene selectivity (C-mol%) | 28.5 | 33.8 | 47.8 |
| | Butene selectivity (C-mol%) | 15.8 | 12.8 | 22.0 |
| | Catalytic life (hr) | 1 | 5 | 7 |

As shown in Table 4, it can be seen that the zeolites according to the Examples having a molar ratio (Si/Al) of silicon (Si) to aluminum (Al) of 200 or more and 2000 or less maintain a high raw material conversion rate for a long period of time. Among these, the zeolite according to Example 2, which used TCDEOH as the raw material for synthesizing the zeolite, showed the longest life.

## Claims

1. A quaternary ammonium salt represented by the following general formula (I) or the general formula (II), 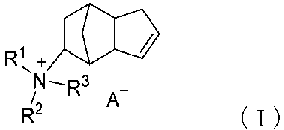 wherein R¹, R², and R³ each independently represent an alkyl group, and A⁻ represents an anion, 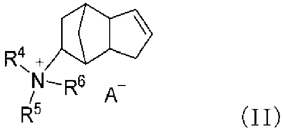 wherein R⁴, R⁵, and R⁶ each independently represent an alkyl group, and A⁻ represents an anion.

2. The quaternary ammonium salt according to claim 1,
wherein the general formula (I) is represented by the following general formula (I-exo) and the general formula (II) is represented by the following (II-exo), 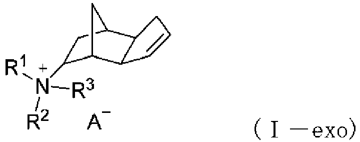 wherein R¹, R², and R³ each independently represent an alkyl group, and A⁻ represents an anion, 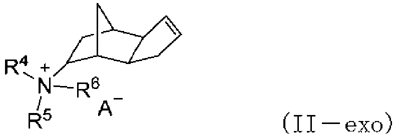 wherein R⁴, R⁵, and R⁶ each independently represent an alkyl group, and A⁻ represents an anion.

3. The quaternary ammonium salt according to claim 1 or 2,
wherein R¹, R², R³, R⁴, R⁵ and R⁶ each independently represent an alkyl group having a carbon number of 1 to 6, and A is OH or a halogen atom.

4. An organic structure-directing agent comprising the quaternary ammonium salt according to claim 1 or 2.

5. The organic structure-directing agent according to claim 4 comprising a quaternary ammonium salt represented by the following general formula (III), 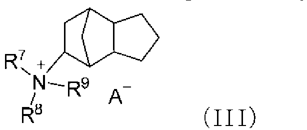 wherein R⁷, R⁸, and R⁹ each independently represent an alkyl group, and A⁻ represents an anion.

6. The organic structure-directing agent according to claim 5,
wherein the general formula (III) is a quaternary ammonium salt represented by the following general formula (III-exo), 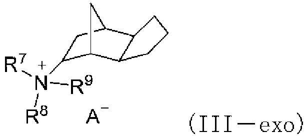 wherein R⁷, R⁸, and R⁹ each independently represent an alkyl group, and A⁻ represents an anion.

7. A method for producing a quaternary ammonium salt represented by at least either of the following general formulas (I) and (II), or the general formula (III),
wherein dicyclopentadiene is used as a raw material, 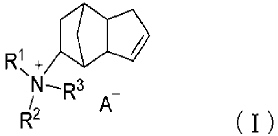 wherein, R¹, R², and R³ each independently represent an alkyl group, and A⁻ represents an anion, 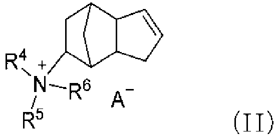 wherein, R⁴, R⁵, and R⁶ each independently represent an alkyl group, and A⁻ represents an anion, 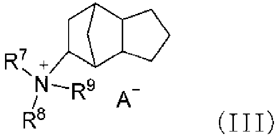 wherein, R⁷, R⁸, and R⁹ each independently represent an alkyl group, and A⁻ represents an anion.

8. The method for producing a quaternary ammonium salt according to claim 7,
wherein R¹, R², R³, R⁴, R⁵, R⁶, R⁷, R⁸ and R⁹ are each independently an alkyl group having a carbon number of 1 to 6, and A is each independently OH, HCO₃, MeCO₃, or a halogen atom.

9. The method for producing a quaternary ammonium salt according to claim 7, comprising the following steps (1) to (4) in order,
step (1): a step of producing 8-hydroxytricyclo[5.2.1.0^{2,6}]decene by hydrating dicyclopentadiene,
step (2): a step of producing 8-ketotricyclo[5.2.1.0^{2,6}]decene by oxidizing the 8-hydroxytricyclo[5.2.1.0^{2,6}]decene obtained in the step (1),
step (3): a step of producing dialkylaminotricyclo[5.2.1.0^{2,6}]decene by aminating the 8-ketotricyclo[5.2.1.0^{2,6}]decene obtained in the step (2),
step (4): a step of producing trialkyltricyclo[5.2.1.0^{2,6}]decene ammonium salt by alkylating the dialkylaminotricyclo[5.2.1.0^{2,6}]decene obtained in the step (3).

10. The method for producing a quaternary ammonium salt according to claim 7, comprising the following step (1), steps (2-2) to (4-2) in order,
wherein the step (2-2) and the step (3-2) are performed by using a solid catalyst,
step (1): a step of producing 8-hydroxytricyclo[5.2.1.0^{2,6}]decene by hydrating dicyclopentadiene,
step (2-2): a step of producing 8-ketotricyclo[5.2.1.0^{2,6}]decane by oxidizing and hydrogenating the 8-hydroxytricyclo[5.2.1.0^{2,6}]decene obtained in the step (1),
step (3-2): a step of producing dialkylaminotricyclo[5.2.1.0^{2,6}]decane by aminating 8-ketotricyclo[5.2.1.0^{2,6}]decane obtained in the step (2-2),
step (4-2): a step of producing trialkyltricyclo[5.2.1.0^{2,6}]decane ammonium salt by alkylating the dialkylaminotricyclo[5.2.1.0^{2,6}]decane obtained in the step (3-2).

11. The method for producing a quaternary ammonium salt according to claim 7, comprising the following step (1), steps (2-2) to (4-2) in order,
wherein the step (2-2) and the step (3-2) are performed by using the same catalyst,
step (1): a step of producing 8-hydroxytricyclo[5.2.1.0^{2,6}]decene by hydrating dicyclopentadiene,
step (2-2): a step of producing 8-ketotricyclo[5.2.1.0^{2,6}]decane by oxidizing and hydrogenating the 8-hydroxytricyclo[5.2.1.0^{2,6}]decene obtained in the step (1),
step (3-2): a step of producing dialkylaminotricyclo[5.2.1.0^{2,6}]decane by aminating 8-ketotricyclo[5.2.1.0^{2,6}]decane obtained in the step (2-2),
step (4-2): a step of producing trialkyltricyclo[5.2.1.0^{2,6}]decane ammonium salt by alkylating the dialkylaminotricyclo[5.2.1.0^{2,6}]decane obtained in the step (3-2).

12. The method for producing a quaternary ammonium salt according to any one of claims 9 to 11, comprising the following step (A),
step (A): a step of ion-exchanging the anion A⁻ of the trialkyltricyclo[5.2.1.0^{2,6}]decene ammonium salt obtained in the step (4) or the trialkyltricyclo[5.2.1.0^{2,6}]decane ammonium salt obtained in the step (4-2).

13. The method for producing a quaternary ammonium salt according to claim 12, wherein the anion is exchanged with OH⁻ by the ion exchange in the step (A).

14. A CON-type zeolite which satisfies the following (1) and (2),
(1) comprising silicon (Si) and aluminum (Al) as constituent elements, wherein a molar ratio (Si/Al) of silicon (Si) and aluminum (Al) is 200 or more and 2000 or less,
(2) which is a mixed crystal of Polymorph A and Polymorph B, and contains 25 mass% or more of Polymorph A.

15. The CON-type zeolite according to claim 14, comprising silicon (Si) and boron (B) as constituent elements,
wherein a molar ratio (Si/B) of silicon (Si) and boron (B) is 5 or more and 100000 or less.

16. The CON-type zeolite according to claim 14,
wherein the zeolite is SSZ-33.

17. The CON-type zeolite according to claim 14, having an average primary particle diameter of 1000 nm or less.

18. The CON-type zeolite according to claim 14,
wherein a ratio (A2/A1) of the BET specific surface area (A2) calculated by BET plot to the external surface area (A1) calculated by t plot in a nitrogen adsorption/desorption measurement, is 10 or less.

19. A method for producing a CON-type zeolite, which uses an organic structure-directing agent containing at least one of the quaternary ammonium salts represented by the following general formulas (I) and (II) as a raw material for zeolite synthesis, 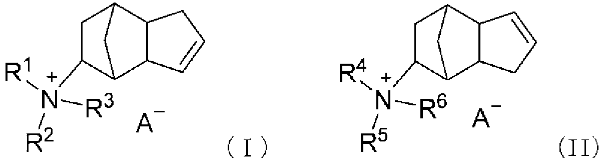 wherein R¹, R²,R³, R⁴, R⁵ and R⁶ each independently represent an alkyl group, and A⁻ represents an anion.

20. The method for producing a CON-type zeolite according to claim 19,
wherein R¹, R², R³, R⁴, R⁵ and R⁶ of the quaternary ammonium salt are all methyl groups, and A is OH.

21. The method for producing a CON-type zeolite according to claim 19,
wherein the organic structure-directing agent contains a quaternary ammonium salt represented by the following general formula (III), 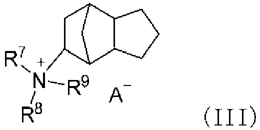 wherein R⁷, R⁸, and R⁹ each independently represent an alkyl group, and A⁻ represents an anion.

22. A catalyst comprising the CON-type zeolite according to any one of claims 14 to 18.

23. The catalyst according to claim 22,
wherein the catalyst is used in a reaction for producing a lower olefin.

24. A method for producing a lower olefin, comprising a step of contacting the catalyst according to claim 23 with a raw material containing methanol or/and dimethyl ether.
